(19) Europäisches Patentamt
European Patent Office
Office européen des brevets

(11) Publication number: 0 260 118 B1

(12) # EUROPEAN PATENT SPECIFICATION

(45) Date of publication of patent specification: 04.12.91

(51) Int. Cl.⁵: C07C 277/00, C07C 279/00, C07K 5/06

(21) Application number: 87307957.8

(22) Date of filing: 09.09.87

(54) **Selective amidination of diamines.**

(30) Priority: 10.09.86 US 905827

(43) Date of publication of application:
16.03.88 Bulletin 88/11

(45) Publication of the grant of the patent:
04.12.91 Bulletin 91/49

(84) Designated Contracting States:
AT BE CH DE ES FR GB GR IT LI LU NL SE

(56) References cited:
EP-A- 0 012 401
EP-A- 0 050 800
EP-A- 0 137 742
EP-A- 0 196 841
GB-A- 1 587 258
US-A- 4 477 429

THE JOURNAL OF ORGANIC CHEMISTRY, vol. 51, no. 10, May 16, 1986, "A Convenient Synthesis of Guanidines from Thioureas" (CYNTHIA A. MARYANOFF et al.)

(73) Proprietor: SYNTEX (U.S.A.) INC.
3401 Hillview Avenue P.O. Box 10850
Palo Alto California 94303(US)

(72) Inventor: Arzeno, Humberto B.
7665 Normandy Way
Cupertino California 95014(US)
Inventor: Morgans, David J. Jr.
731 Madrone Avenue
Sunnyvale California 94086(US)

(74) Representative: Armitage, Ian Michael et al
MEWBURN ELLIS & CO. 2/3 Cursitor Street
London EC4A 1BQ(GB)

EP 0 260 118 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the selective amidination of diamines, and especially to ω-guanidino-α-amino acids, and to the preparation of these acids, their functional derivatives, and compounds, such as peptides, containing them.

European Patent Application number 196,841 filed 21 March 1986, describes a number of $N^G,N^{G'}$-dialkylguanidino dipeptides, which are stated to be useful as angiotensin-converting enzyme (ACE) inhibitors. These dipeptides may be considered to contain the modified amino acids $N^G,N^{G'}$-dialkylarginine, $N^G,N^{G'}$-homoarginine, and $N^G,N^{G'}$-dihomoarginine; and may be prepared by a number of methods, which include (1) the reductive condensation of one of these amino acids with an α-ketoamide to form the dipeptide, and (2) the alkylation of a precursor dipeptide containing an ω-amine group, e.g. a dipeptide based on ornithine, lysine, or homolysine, with an alkylating formamidine derivative (an amidinating agent). Both cf these synthetic methods involve the amidination of an ω-amine group with an alkylating formamidine derivative such as a haloformamidine or an (alkylthio)formamidine (an S-alkylisothiourea): in (1) in the preparation of a starting material, and in (2) in the reaction itself, and are discussed in the above-mentioned European Patent application, as are the syntheses of the starting materials.

A synthesis of ethyl $N^G,N^{G'}$-diethyl-L-homoarginate hydrochloride is disclosed in J.J. Nestor, Jr., et al., "Peptides - Structure and Function", Proc. Eighth Amer. Peptide Symposium, V.J. Hruby and D.H. Rich, Eds., Pierce Chem. Co., Rockford IL, 1984, pp. 861-4. However, the use of an S-alkylisothiourea as an alkylating agent is attended with two disadvantages: first, the yield of the desired ω-guanidino acid is low; and second, the byproduct of the reaction is a noxious alkyl mercaptan. If an S-methylisothiourea is chosen, as is commonly done, methyl mercaptan (a gas detectable at a concentration of 1 ppb), results.

W. Walter, Angew. Chem., 67, 1955, p. 275, describes the reaction of formamidinesulfinic acid with glycine under basic conditions to yield 35% of N-(aminoiminomethyl)glycine, an α-guanidino acid. British Patent No. 1,587,258 (Aktieselskabet Gea) describes the preparation of guanidines by the reaction of ammonia and primary amines with formamidinesulfonic acids, which may be mono- or di-substituted with alkyl or phenylalkyl groups. C.A. Maryanoff et al., J. Org. Chem., 51, 1986, pp. 1882-4, describe the reaction of mono(alkyl or aryl)formamidinesulfonic acids with primary and secondary amines to prepare guanidines.

However, none of these publications shows the amidination of an α,ω-diamino acid or a functional derivative thereof with a formamidinesulfonic acid.

This invention relates to the selective amidination of diamines, in particular to the preparation of an ω - guanidino-α-amino acid or its functional derivative, or a compound, such as a peptide, containing it, by the reaction of an α,ω-diamino acid or its functional derivative, or a compound containing it, with a formamidinesulfonic acid.

Peptides that contain an ω-guanidino-α-amino acid or its functional derivatives may be prepared by a number of methods, which include (1) the coupling of an ω-guanidino-α-amino acid or its functional derivative with an amino acid or a functional derivative thereof to form the peptide, and (2) the alkylation of a precursor peptide containing an ω-amino group, e.g. a peptide based on ornithine, lysine, or homolysine, with an alkylating formamidinesulfonic acid derivate (an amidinating agent).

Both of these synthetic methods involve the amidination of an ω-amino group with an alkylating formamidinesulfonic acid derivative: (1) in the preparation of a starting material for the synthesis of guanidino peptides, and (2) in the preparation of guanidino peptides. These two methods are discussed in more detail in the "Preparation of Compounds of Formula III" section of this disclosure.

According to the present invention there is provided a method for preparing a compound of the formula:

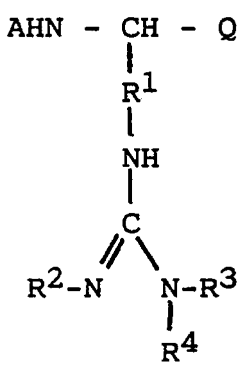

(III)

or the pharmaceutically acceptable, non-toxic salts thereof, in which

A is H, R (wherein R is: optionally substituted lower alkyl, namely branched or unbranched alkyl of from 1 to 6 carbon atoms wherein one or more hydrogen atoms is optionally substituted by lower alkyl of from 1 to 3 carbon atoms, lower alkoxy of from 1 to 3 carbon atoms, halogen or trifluoromethyl; phenyl wherein one or more hydrogen atoms is optionally substituted by lower alkyl of from 1 to 3 carbon atoms, lower alkoxy of from 1 to 3 carbon atoms, halogen or trifluoromethyl; or aralkyl selected from phenyl-substituted lower alkyl of from 1 to 3 carbon atoms wherein one or more hydrogen atoms is optionally substituted by lower alkyl of from 1 to 3 carbon atoms, lower alkoxy of from 1 to 3 carbon atoms, halogen or trifluoromethyl), R-O-CO-, peptidyl residue, or (peptidyl residue)-CO-(lower alkyl as defined above);

Q is a carboxylate-related group;

$R^1$ is optionally substituted lower alkylene namely an unbranched saturated hydrocarbon chain containing from 2 to 6 carbon atoms wherein one or more hydrogen atoms is optionally substituted by lower alkyl of from 1 to 3 carbon atoms, lower alkoxy of from 1 to 3 carbon atoms, halogen or trifluoromethyl; and

$R^2$, $R^3$, and $R^4$ are each independently H or R (as defined above), with the proviso that at least one of $R^2$, $R^3$, and $R^4$ is not hydrogen,

which method comprises

(a) alkylating a compound of the formula:

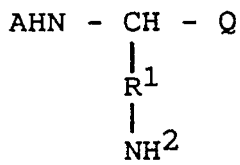

(I)

or a functional derivative thereof in which

A, Q, and $R^1$ are as previously defined with a formamidinesulfonic acid of the formula:

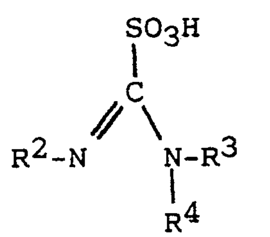

(II)

in which

$R^2$, $R^3$, and $R^4$ are as previously defined and optionally:

(b) converting an acid addition salt of the compound of formula (III) with base to the compound of formula (III) or a salt thereof with said base; or

(c) converting a salt of the compound of formula (III) (derived from a base) with acid to the free compound of formula (III) or an acid addition salt thereof; or

(d) converting a salt of the compound of formula (III), preferably a soluble salt, to another salt of the compound of formula (III), preferably less soluble than said soluble salt.

Compounds of the formula III, prepared according to methods described herein, may be useful for the inhibition of angiotensin converting enzyme (ACE).

A further aspect of the invention is the use of the compounds of formula (III) for the preparation of ACE-inhibitors.

## Definitions

As used herein, "lower alkyl" means a branched or unbranched saturated hydrocarbon chain containing from one to six carbon atoms, such as methyl, ethyl, isopropyl and n-hexyl;

"lower alkoxy" means the group -OR, where R is lower alkyl as defined above, such as methoxy, ethoxy, isopropoxy, and n-hexyloxy;

"lower alkylene" means an unbranched saturated hydrocarbon chain containing from two to six carbon atoms, such as ethylene, 1,3-propylene, and 1,5-pentylene;

"optional" or "optionally" means that the subsequently described event or circumstance may or may not occur, and that the description includes instances where the event or circumstance occurs and where it does not;

"optionally substituted", when applied to a compound or part thereof, means that one or more of the hydrogen atoms may be substituted by lower alkyl or lower alkoxy, both having one to three carbon atoms, halogen, or trifluoromethyl;

"aralkyl" means an optionally substituted phenyl-substituted lower alkyl of one to three carbon atoms, such as benzyl, 4-methoxybenzyl, and 2-phenylethyl;

"ω" defines the position of a group that is at least three contiguous carbon atoms removed from Q. For example, the amino group, the guanidino group or the group of the formula

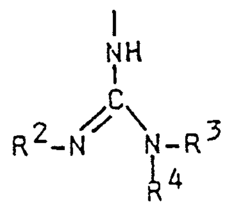

wherein $R^2$, $R^3$, and $R^4$ have the above meanings;

"functional derivative", when applied to α,ω-diamino acids, refers for example, to derivatives of the carboxylic acid group or the α-amino group, i.e. an amide (as, e.g., in a peptide), ester, nitrile, or acid halide, in particular to the optionally substituted amide, ester, or nitrile (i.e. where -Q of compound (I) is $-CONR^{11}R^{12}$, $-COOR^{13}$, or -C≡N, in which each of $R^{11}$ and $R^{12}$ is selected from H and R (R being optionally substituted lower alkyl, phenyl, and aralkyl), and $R^{13}$ is R). The term includes the case where the carboxylate group of the α,ω-diamino acid forms a peptide bond with the α-amine group of another amino acid or peptide, i.e. where the hydroxy portion of the carboxylate group is replaced by a peptidyl residue (preferably wherein the peptidyl residue contains an un-natural cyclic imino acid). The term also refers to replacement of one of the hydrogens of the α-amine group to form an amide (e.g. a peptide) or amine bond to the nitrogen, e.g. where A of compound (I) is R, R-O-CO-, a peptidyl residue, or (peptidyl residue)-CO-(lower alkyl)- (preferably wherein the peptidyl residue contains an un-natural cyclic imino acid). Preferred configurations of the (peptidyl residue)-CO-(lower alkyl)- group have the formula;

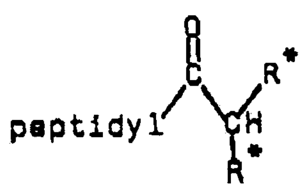

wherein R* is an alkyl group of one to five carbon atoms. The term further refers to a derivative of the amino acid wherein both the -COOH and $H_2N$- groups are functionalized as immediately previously described;

"carboxylate-related group" refers to a carboxylic acid group, a carboxylate group or a functional derivative of the carboxylic acid group as defined above.

"pharmaceutically acceptable, non-toxic salts" means those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, menthanesulfonic acid, ethanesulfonic acid, p-toluenesulfonic acid, and salicylic acid.

The compounds of formula III also form salts with inorganic bases such as sodium hydroxide, potassium hydroxide, calcium hydroxide, aluminum trihydroxide, magnesium hydroxide or with organic bases such as isopropylamine, N-methylglucamine, glucamine, trimethylamine, diethylamine, ethanolamine, 2-dimethylaminoethanol or tromethamine.

"amino acid" means the natural and un-natural α-amino acids and derivatives thereof. Preferred natural

amino acids include the aliphatic amino acids such as alanine and leucine, hydroxyamino acids such as serine and threonine, dicarboxylic amino acids and their amides such as aspartic acid and glutamine, amino acids having basic functions such as hydroxylysine, lysine, arginine and histidine, aromatic amino acids such as phenylalanine, tyrosine and tryptophan, sulfur-containing amino acids such as cysteine and methionine, and imino acids such as proline and hydroxyproline. Preferred un-natural amino acids include the cyclic imino acids derived from proline such as 3-carboxy-1,2,3,4-tetrahydro-isoquinoline (THIQ), 2-carboxy-2,3-dihydro-indole, and 4,5-trimethylene proline (which is the same as 3-carboxy-2-aza-bicyclo-[3.3.0]-octane).

"peptidyl residue" means an amino acid (or a derivative thereof) or a peptide (or a derivative thereof) which is bonded to a -CO- or -NH- group (not part of the peptidyl residue) by an amide (peptide) bond;

"water-miscible organic solvent" includes (lower alkyl) alcohols (e.g. methanol) and ethers (e.g. tetrahydrofuran, dioxane, and dimethoxyethane); and

"polar organic solvent" includes solvents such as acetonitrile.

## Starting Materials

The diamino acids of compound (I) of this invention are generally commercially available, or may readily be prepared by methods known to those of ordinary skill in the art. Preferred diamino acids are those where the alkylene-group contains from three to five carbon atoms, i.e. ornithine, lysine, and homolysine; and a particularly preferred diamino acid is lysine. The diamino acids are asymmetric, and may be available as (R)-, (S)-, and (R,S)-acids. The present method is equally applicable to each form, and does not cause racemization at the α-carbon.

Functional derivatives of the diamino acids constituting compound (I) of this invention may be readily prepared from the diamino acids by methods known to those of ordinary skill in the art, e.g. esterification of the carboxylate group with alcohols, protection of the α-amine group with t-butoxycarbonyl, etc. ; and a number of functional derivatives, such as esters and Nα-protected compounds, are commercially available, e.g. from Sigma Chemical Company or other biochemical suppliers. Preferred functional derivatives of the diamino acids constituting compound (I) of this invention, other than peptides, are the esters of the acids, especially the lower alkyl esters, and the Nα-protected derivatives of the acids and esters, such as the Nα-t-butoxycarbonyl acids and esters.

Peptides containing the diamino acids constituting compound (I) of this invention may be prepared by methods of peptide synthesis known to those of ordinary skill in the art or can be prepared by methods described in European Patent Publication Numbers 12401, 65301, 79022, 196,841; and a number of peptides are commercially available. Preferred peptides are (S)-2-[(S)-N-[(S)-1-carboxyl-5-aminopentyl]-alanyl]-1,2,3,4-tetrahydroisoquinoline-3-carboxylic acid and its esters, and the similar peptides disclosed as precursors to the $N^G,N^{G'}$-dialkylguanidino peptides in European Patent Publication Number 196,841, referred to previously.

## Preparation of Compounds of Formula II

Compounds of formula II may be prepared by the method of C.A. Maryanoff et al., J. Org. Chem., 51, 1986, pp. 1882-4, for sulfonic acids from thioureas (or by the references cited therein for that purpose), or by the following modification of that procedure. The starting thioureas are generally commercially available, or may readily be prepared by methods known to those of ordinary skill in the art. Preferred thioureas are those of the formula $R^2HN-C(S)-NR^3R^4$ where each of $R^2$ and $R^3$ is R, and $R^4$ is H; more preferred of those are where each of $R^2$ and $R^3$ is optionally substituted lower alkyl, and $R^4$ is H; and particularly preferred is 1,3-diethyl-2-thiourea. Other preferred thioureas are those where $R^2$ is optionally substituted phenyl and each of $R^3$ and $R^4$ is hydrogen, e.g. 1-phenyl-2-thiourea. The thiourea and an alkali molybdate (0.001 - 0.1 equivalents, with 0.002 - 0.01 equivalents being preferred) are dissolved or suspended in water, optionally containing a water-miscible organic solvent, and cooled to 0 - 15° C, preferably 0 - 5° C. The solution is stirred vigorously at that temperature, and approximately 3 equivalents of hydrogen peroxide are added dropwise sufficiently slowly to maintain the temperature below 20° C, preferably below 10° C (typically, 30 minutes - 4 hours, usually about 1 - 2 hours, is required). The solution is stirred for a further 30 minutes - 4 hours, usually about 1 - 2 hours at that temperature, then allowed to warm to room temperature and stirred at room temperature for 10 - 30 hours. Excess oxidant is destroyed by cooling the solution to 0 - 20° C, preferably 0 - 10° C (to minimize decomposition of the product), and adding a sufficient quantity of a reducing agent such as an alkali bisulfite until the solution becomes colorless or a test for peroxide is negative. The solution of the sulfonic acid may be filtered and used directly, or the acid may be isolated, if

desired. If it is desired to isolate the acid, a relatively smaller amount of water may be used initially (e.g. 0.5 mL/mmol thiourea), and the acid may be isolated by methods such as removal of the solvent, e.g. by lyophilization of the solution, followed by dissolution of the solid acid in a lower alkyl alcohol (e.g. methanol) and precipitation with, e.g. diethyl ether; if it is desired to use the sulfonic acid directly, a relatively larger volume of water (e.g. 1.2 mL/mmol thiourea) is preferred.

Preparation of Compounds of Formula II

The method of preparation of the desired compound of formula III is largely dependent upon the properties of the precursor compound of formula I, e.g. whether the compound of formula I is water-soluble. Since the reaction of compounds of formula I with formamidinesulfonic acid is relatively slow, conditions may be chosen to maximize the formation of the ω-guanidino derivative while minimizing formation of the α,ω-bis(guanidino) derivative. For example, the reaction may, but need not, be carried out in the presence of an auxiliary base soluble in the reaction solvent. If an auxiliary base is not used, it is preferred that the compound of formula I be present in excess over the formamidinesulfonic acid, as excess sulfonic acid would protonate the ω-amine group and render it less reactive. If an auxiliary base is used, an excess of the formamidinesulfonic acid may be employed to increase the rate of reaction; however, when the α-amine of the compound of formula I is unsubstituted, the excess should be small to avoid formation of the α,ω-bis-(guanidino) derivative. Similarly, a wide range of reaction temperatures may be employed, from -10 °C to as high as reflux temperature of the solvent, typically between about 0 °C and room temperature, provided that these are otherwise consonant with the compound of formula I, e.g. they do not cause racemization at asymmetric carbon atoms, etc. The general reaction conditions described in the Maryanoff et al. article previously referred to may be taken , or the methods described in the following paragraphs and the Examples may be employed as a basis for suitable reaction conditions.

When the compound of formula I is water-soluble, e.g. when the compound of formula I is a diamino acid, a non-esterified peptide, or the like, the following method is suitable. The compound of formula I is dissolved in sufficient aqueous strong base (suitable strong bases include sodium hydroxide, potassium hydroxide, and the like) to give a solution of pH about 10 - 12, preferably 10.5 - 11. This solution is vigorously stirred, and maintained at that pH (e.g. by the addition of appropriate amounts of concentrated aqueous strong base) while the appropriate sulfonic acid is added, e.g. by addition of a solution prepared as above. The addition may take place over 1 - 72 hours, typically 6 - 24 hours. When the addition is completed, the reaction mixture is stirred for 5 to 25 hours, at a temperature about room temperature if the addition has taken place under cooling. The total reaction time, ratio of reactants, presence of base, etc., may be varied depending on the relative reaction rates at the α- and ω-nitrogen atoms. The product of formula III may then be isolated by conventional methods or by the methods disclosed in European Patent Publication Numbers 12401, 65301, 79022 and 196,841. Alternatively, the product may be carried in solution into another reaction, e.g. the ω-guanidino-α-amino acid may be protected, as by formation of the Nα-t-butoxycarbonyl derivative, and the resulting t-BOC guanidinoamino acid isolated.

If the compound of formula I is not sufficiently soluble in aqueous base (and it is generally desirable that the solution of compound I not be too dilute, as otherwise the reaction would be excessively slow), the aqueous base of the previous paragraph may be replaced by a solution of a base in a mixture of water and a water-miscible organic solvent, e.g. a water/methanol mixture.

If the compound of formula I is of low solubility in aqueous base, e.g. when the compound lacks ionizable amine or carboxylate groups, such as a peptide diester, a polar organic solvent in which the sulfonic acid is also soluble may instead by used. A preferred solvent is acetonitrile, and preferred bases, when employed, are non-reactive organic bases at least as basic as triethylamine, e.g. tertiary amines such as triethylamine, N-methylpiperidine, and the like. The compound of formula I is dissolved in the solvent, and the sulfonic acid (as a solid or in solution) added with vigorous stirring. The reaction conditions are similar to those given above for aqueous solvents, and the comments above are applicable to non-aqueous solvents.

The procedures described above are particularly applicable to the preparation of angiotensin converting enzyme inhibitors of the formula A

$$R^5 - CO - \underset{\displaystyle R^6}{\underset{|}{CH}} - NH - \overset{\displaystyle R^7}{\overset{|}{CH}} - CO - \overset{\displaystyle R^8}{\overset{|}{N}} - \overset{\displaystyle R^9}{\overset{|}{CH}} - CO - R^{10} \qquad (A)$$

wherein $R^5$ represents hydroxy, lower alkoxy, benzyloxy or amino optionally substituted with one or two lower alkyl groups;

$R^6$ and $R^7$ represent

(1) hydrogen or preferably lower alkyl (optionally omega-substituted with phenyl or naphthyl, less preferred omega-substituted with di(lower)alkylguanidino), or

(2) 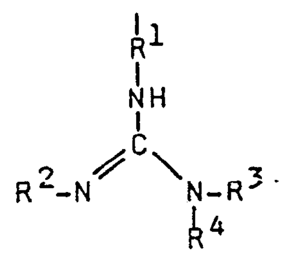

wherein $R^1$, $R^2$, $R^3$ and $R^4$ have the above meanings; and one of $R^6$ and $R^7$ being (1), the other being (2); and

the group -

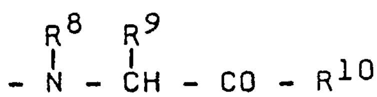

is an amino acid residue selected from the group of imino acid residues, preferably cyclic amino acid residues in which the subgroup

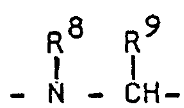

forms a heterocyclic radical containing one nitrogen atom and up to 9 ring carbon atoms, in particular said cyclic imino acid residue is or is derived from proline, hydroxyproline or proline analogs;

$R^{10}$ is hydroxy, lower alkoxy, benzyloxy or amino optionally substituted with one or two lower alkyl groups; and their pharmaceutically acceptable, non-toxic salts.

A specific process in accordance with the present invention which utilize the described reaction [(I) + (II) → (III)] as the last step comprises:

(a) alkylating a peptide of the formula

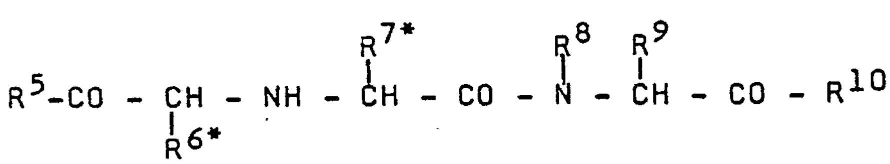

wherein one of $R^{6*}$ and $R^{7*}$ is - $(CH_2)_nNH_2$ and the other of $R^{6*}$ and $R^{7*}$ is (1), preferably (S)-2-[(S)-N-[(S)-1-carboxy-5-aminopentyl]alanyl]-1,2,3,4-tetrahydro-isoquinoline-3-carboxylic acid and its esters, with an alkylating agent of the formula

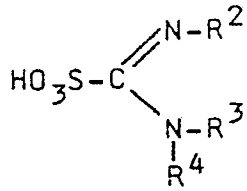

wherein $R^2$, $R^3$ and $R^4$ are as previously defined.

Additional processes that utilize compounds prepared by the method of the present invention (compounds of the general formula (III)) for the preparation of compounds of the formula A are as follows:

(b) condensing an amino acid derivative of the formula

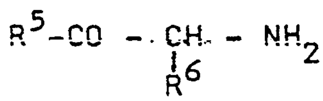

wherein $R^6$ is the group (2) described above, with an α-ketoacid derivative of the formula

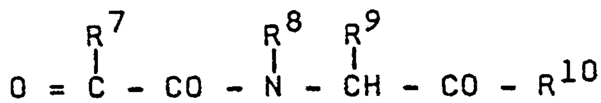

under reduction conditions to form a compound of formula (A);

(c) condensing an α-ketoacid derivative of the formula

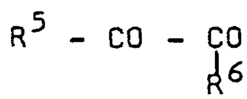

with a peptide of the formula

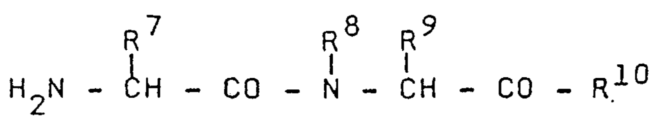

wherein $R^7$ is the group (2) described above, under reductive conditions to form a compound of formula (A);

(d) condensing an amino acid derivative of the formula

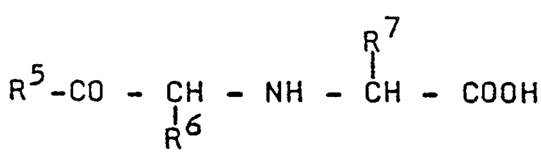

with proline or a proline analog derivative of the formula

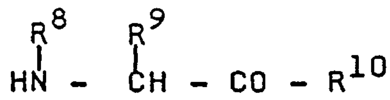

to form a compound of formula A;
(e) alkylating a peptide derivative of the formula

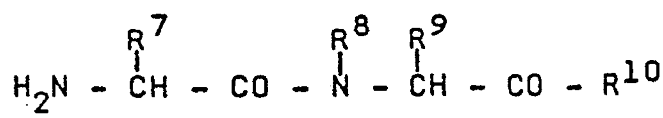

wherein $R^7$ is the group (2) above,
with an alkylating agent of the formula

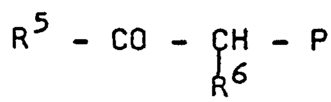

wherein P is halo selected from the group of chloro, bromo or iodo, or a sulfonyloxy group, to form a compound of formula A;
(f) alkylating an amino acid derivative of the formula

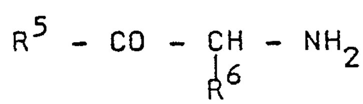

wherein $R^6$ is the group (2) above,
with an alkylating agent of the formula

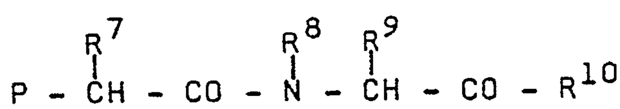

wherein P has the meanings of step (e), to form a compound of formula A;
(g) hydrolyzing a compound of the formula

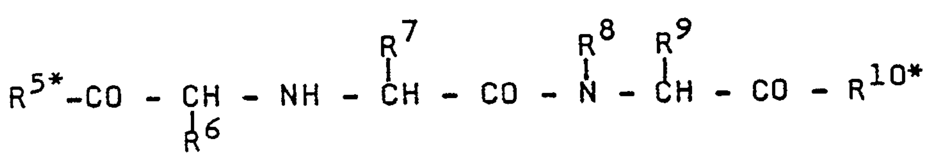

wherein $R^{5*}$ and $R^{10*}$ are hydroxy, lower alkoxy or benzyloxy and at least one of $R^{5*}$ and $R^{10*}$ is lower alkoxy or benzyloxy to a compound of formula A wherein $R^5$ and $R^{10}$ are hydroxy;
(h) hydrogenolyzing (reductively cleaving) a compound of the formula

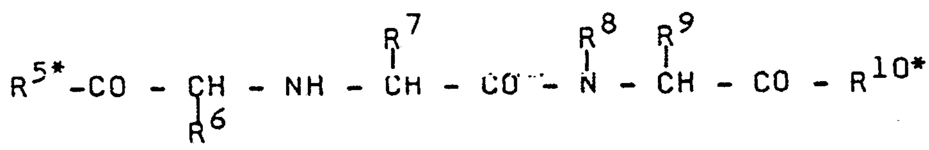

wherein $R^{5*}$ and $R^{10*}$ are hydroxy, lower alkoxy or benzyloxy and at least one or $R^{5*}$ and $R^{10*}$ is benzyloxy to a compound of formula A wherein at least one of $R^5$ and $R^{10}$ is hydroxy;

(i) reacting a compound of formula A with a base to form a salt of the compound of formula A;

(j) reacting a compound of formula A with an acid to form an acid addition salt of the compound of formula A;

(k) esterifying a compound of the formula

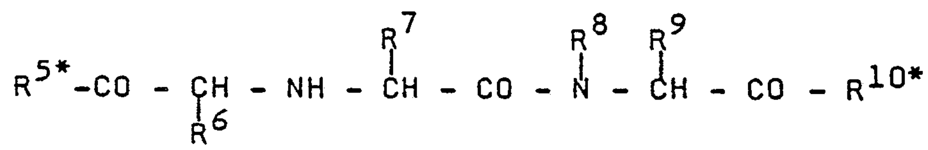

wherein at least one of $R^{5*}$ and $R^{10*}$ is hydroxy and the other is hydroxy, lower alkoxy or benzyloxy with a lower alkanol or benzyl alcohol;

(l) acylating ammonia, a lower alkyl amine or a di(lower)alkyl amine with a dipeptide derivate of the formula

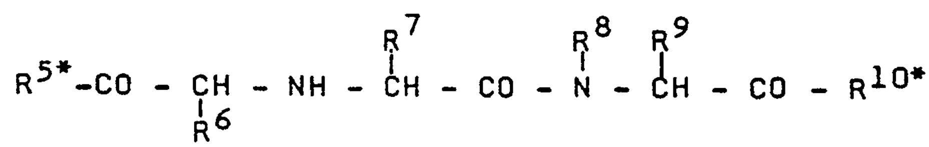

wherein $R^{5*}$ and $R^{10*}$ are hydroxy, chloro, bromo, lower alkoxy, benzyloxy, a lower alkanoyloxy group, or amino optionally substituted with one or two lower alkyl groups, and at least one of $R^{5*}$ and $R^{10*}$ is hydroxy, chloro, bromo, lower alkoxy, benzyloxy, or a lower alkanoyloxy group.

(m) converting an acid addition salt of the peptide of formula A with base to the peptide of formula A or a salt thereof with said base;

(n) converting a salt of a peptide of formula A (derived from a base) with acid to the free peptide of formula A or an acid addition salt thereof; and

(o) converting a salt of the peptide of formula A, preferably a soluble salt to another salt of the peptide of formula A, preferably less soluble than said soluble salt.

In alkylation steps (a), (e) and (f) an amino acid derivative, or a peptide with a free amino group, are alkylated with an appropriate alkylating agent.

In step (a) the peptide is alkylated according to the method of the present invention with a formamidinesulfonic acid derivative. The reaction is carried out according to the reaction general reaction conditions described above.

In step (e) a peptide is dissolved in an appropriate solvent and is alkylated with the appropriate α-haloacid (ester or amide) or a α-sulfonyloxy acid (ester or amide) under basic conditions in water or an organic solvent wherein the peptide contains an alkylated guanidino group prepared according to the method of the present invention. The temperature will be kept between 0° and 70°C, preferably at about room temperature. The α-halogen is chloro, bromo, or iodo or P can be a lower alkylsulfonyloxy or a aryl(6 to 12C)sulfonyloxy group.

In step (f) the alkylating agent is an α-halo-acyl or α-sulfonyloxyacyl amino acid derivative and the reaction will be conducted under basic conditions in water or in another solvent containing the substrate to be alkylated and adding the alkylating agent. The reaction temperature will be kept between -20° and 70°C, preferably at about room temperature. The nature of the acyl group depends on the choice of the substituent $R^7$. For example, α-chloro- or α-bromoacetylated aminoacids can be used as alkylating agents. The substrate to be alkylated (amino acid or a derivative thereof) contains an alkylated guanidino group prepared according to the methods described herein.

The alkylating agent of step (f) may be in the form of the free acid, its ester, or its amide. The amino acid or peptide derivative in all these alkylating steps may also be present in the form of their esters or amides.

Step (b) and step (c) involve reductive condensations. In both steps an α-ketoacid derivative is condensed with a free amino group (of an amino acid in step (b) or of a peptide in step (c)). In both steps, the fragment containing the amino group (an amino acid or a derivative thereof or a peptide) contains an alkylated guanidino group prepared according to the present invention. The reaction is carried out either in an aqueous solution, optimally in a roughly neutral aqueous solution, or in a suitable organic solvent. The following solvents are suitable, for example nitriles such as acetonitrile, propionitrile, cyclic ethers such as THF or dioxane, lower alkanols such as methanol, ethanol, isopropanol, hydrocarbons such as benzene, toluene and the like or mixtures of these solvents. The reaction conditions correspond to those of a Schiff condensation.

In order to reduce the intermediate Schiff base directly which is the condensation product, the reaction can be carried out in the presence of a reducing agent such as a borohydride complex, for example, sodium cyano borohydride. The reaction proceeds between 0° C and 40° C, preferably at about room temperature.

It is understood that in condensation step (b) and (c) under reductive conditions the α-ketoacid derivative can be the free acid or its ester. The amino acid derivative or the peptide derivative can be in the form of the free acid or of the ester, amide or hydroxamic acid.

Step (d) constitutes the classical formation of the peptide bond. This may be accomplished through activation of the carboxyl group in several fashions. The principal pitfall to be avoided is racemization of the amino acids during the reaction. Racemization yields several products that are frequently very difficult to separate. Suitably activated forms of the carboxyl group include carboxylic acid chlorides (where, however, sometimes racemization occurs), nitro-substituted (6 to 12C)aryl esters, in particular p-nitrophenyl esters, cyanomethyl esters and thioesters. In each case these activated derivatives react smoothly with the amino function or with the imino function of proline, a proline derivative, a proline analog or its derivatives.

In addition, other methods are available that avoid the synthesis of carboxyl-activated intermediates. Preferably the methods involve carbodiimides, such as dicycloalkyl(3-7C)carbodiimides, in particular dicyclohexylcarbodiimide (DCCI), as dehydrating agents.

In general, the condensation reaction conditions will correspond to those used in methods known in the art applied to compounds sensitive to temperature and racemization.

Step (g) involves the hydrolysis of one or both lower alkoxy or benzyl esters of the substructures $R^5$-CO- or -CO-$R^{10}$. Any conventional hydrolytic conditions may be employed which do not affect the peptide bond -CO-N($R^8$)- or racemize the dipeptide in case diastereomeric products are the desired end products. The hydrolysis of step (g) is carried out under acid or basic conditions and leads to the diacid form, which is thought to be the biologically active form. The reaction temperature for the the hydrolysis will be in the range of 0° to 70° C and the reaction will be carried out in an inert solvent containing water or in water. In general, the acid employed will be between 0.1 and 2 normal. If the reaction is carried out under basic conditions, the solution will be 0.1 to 2 normal with respect to the base employed. If the reaction is carried out under basic conditions, in order to obtain the diacid, the reaction mixture will have to be acidified with acid, such as hydrochloric, acetic or sulfuric acid. The acidification will be carried out between -20° and 40° C, since the peptide bond is sensitive to temperature, particularly if the dipeptide is present in enantiomeric form. Temperatures between -20° and 30° C are preferred.

Appropriate acids for the acid hydrolysis of Step (g) are inorganic and organic acids. The following inorganic acids can be used: hydrochloric acid, hydrobromic acid, sulfuric acid, nitric acid, phosphoric acid, and the like.

The following organic acids can be used for the acid hydrolysis: trifluoroacetic acid, trichloroacetic, citric or oxalic acid, and the like.

The following inorganic bases can be used for the basic hydrolysis: sodium hydroxide, potassium hydroxide, calcium hydroxide and the like. The following organic bases can be employed for the basic hydrolysis: trimethylamine, triethylamine, dicyclohexylamine and the like.

The amides of formula A can be hydrolyzed in a manner known per se to the dipeptides of formula A in water or inert solvents containing water using bases or acids as catalysts. The reaction proceeds between -20° to 100° C.

Step (h) involves the hydrogenolysis of benzyl esters. It is self-evident that hydrogenolysis is the method of choice for the production of the two monoesters of Formula A: the compounds wherein $R^5$ is hydroxy and $R^{10}$ is lower alkoxy and the compounds in which $R^5$ is lower alkoxy and $R^{10}$ is hydroxy because the hydrogenolysis will not remove the lower alkoxy groups. The monoesters are thought to be

pro-drugs.

The hydrogenolysis is generally carried out catalytically by hydrogen in the presence of a noble metal, preferably on a carrier, for example, palladium or platinum or rhodium on a carrier such as carbon, or by hydrogen in the presence of Raney nickel. The inert carrier such as carbon will generally contain 5 to 30, preferably 10 percent of the noble metal, in particular palladium. The reaction temperature will be between 0° and 40° C, preferably at about room temperature, under atmospheric pressure or super-atmospheric pressure (up to 10 atm). The reaction times generally range between 30 minutes to 24 hours.

Esterification step (k) is carried out by classical esterification procedures. In the reaction lower alkanols or benzyl alcohol react with the dipeptide of formula A in the presence of strong inorganic or organic acids under the removal of water. The following inorganic acids can be used: hydrochloric acid, hydrobromic acid, sulfuric acid, and the like. The following organic acids can be used: trifluoroacetic acid, sulfonic acids such as perfluorosulfonic, toluenesulfonic acid, and the like. However, the esters of formula A can also be produced using diazoalkenes in particular diazomethane which will give the methyl ester. The reaction will usually be carried out in an inert solvent. Another method that can be used is the reaction of a metallic salt of a compound of formula A which reacts with a lower alkyl or benzyl halide to form a corresponding lower alkyl or benzyl ester. The sodium or cesium salt of the dipeptide or other metal salts can be used to obtain the ester in good yields. In addition, salts of tertiary amines such as 1,8-diazabicyclo [5.4.0]undec-7-ene, or tri(lower)alkyl amines, in particular, triethylamine can employed because of their better solubility.

In another modification of step (k) the esterification is carried out by reacting an acyl halide or diacyl halide of a compound of formula A with a lower alkanol or with benzyl alcohol. This leads to a rapid formation of the ester. In the reaction, 1 mole of the acyl halide is reacted with at least 2 moles of the lower alkanol or benzyl alcohol and the reaction proceeds smoothly to obtain the corresponding diester. In a preferred embodiment the reaction is facilitated by the formation of an adduct with thionylchloride and the lower alkanol or benzyl alcohol at temperatures below 0° C which reacts quickly to form the ester.

In another modification of the process a mixed acid anhydride of the dipeptide of formula A is reacted with an alkanol or benzyl alcohol. The reaction is catalyzed by tertiary organic bases such as pyridine.

The amides of the peptides of formula A [step (1)] in which at least one of $R^5$ and $R^{10}$ is the amino group optionally substituted with one or two lower alkyl groups are prepared by common methods known for the preparation of amides. If only one of $R^5$ and $R^{10}$ is an optionally substituted amino group the compounds are monoamides, whereas if both $R^5$ and $R^{10}$ are an optionally substituted amino group these dipeptides are diamides.

Ammonia or lower alkyl or dialkyl amines can be reacted directly with derivatives of the formula A in particular lower alkyl esters or acid chlorides or acid anhydrides which function as acylating derivatives. Ammonia and the lower alkyl amines (primary amines) and the di(lower alkyl)amines (secondary amines) react very smoothly with these acylating agents. With anhydrides the reaction can be even conducted in aqueous solution. The reaction with acyl halides requires two moles of amine or ammonia, only one of which will be acylated because the second mole combines with the hydrogen halide formed in the reaction. If the acylating agent is an anhydride only one mole of amine or ammonia can be used because the free acid formed is a weak acid and the salt of a weak base and the weak acid dissociates to produce the acylated amine when heated with an excess of the anhydride. Therefore all the amine can be converted into amide by this procedure. If esters are used as acylating agents again the amine and the ester acylation will be reacted in an approximate molar ratio of one to one. The acylation can be conducted in non-acqueous solutions, i.e., in inert solvents such as DMF, lower alkanols such as methanol (or ethanol), and the like. The reaction temperatures will be between -20° and +40° C preferably at about 35° C.

The conversion steps (m), (n) and (o) are usually carried out in inert solvents at temperatures between -20 and 50° C, preferably between -10 and 30° C. In step (m) the acid addition salt of the peptide (which may be mono-or dibasic) is treated with the appropriate equivalent amount of base to form the monobasic salt, the free peptide or to form its salt with the base. In step (n) the peptide sat with base is the starting material and treated with the appropriate equivalent of acid to form the monobasic salt, the free peptide or the acid addition salt (which contains one or two protonated nitrogens).

In step (o) a salt of a peptide is reacted with an inorganic or organic salt to precipitate a less soluble salt (double decomposition). Alternatively, this exchange can be carried out with appropriately charged ion exchange resins.

It is understood that the free peptides of formula A exist in a zwitterionic form, any carboxylic groups being dissociated ($-COO^-$) and the imino group (s) or the $=N-R^2$ group being associated with a proton.

The peptides of formula A or their precursors can also be produced using the known solid-phase technique, in particular using as solid phase polyamide resins that are swellable by polar solvents such as water. After completion of the synthesis the peptide will be removed from the polyamide by hydrolysis,

hydrogenolysis, or transesterification.

The starting materials of steps (a) - (o) are either known from the literature, can be made by known methods from known starting materials (e.g. European Patent Applications 12401, 65301 and 79022) or can be prepared according to the "Starting Materials" section of this disclosure.

The compounds of the formula A are ACE inhibitors and thus block the conversion of the decapeptide angiotensin I to angiotensin II, a highly potent pressor substance. Thus, ACE inhibitors can lower blood pressure by inhibiting the biosynthesis of angiotensin II, especially in animals and humans whose hypertension is angiotensin II related. Furthermore, ACE degrades the vasodilating substance bradykinin. Therefore ACE inhibitors may lower blood pressure by potentiating bradykinin's effects, as well as by inhibiting angiotensin II. Although the relative importance of these and other possible mechanisms remains to be established, ACE inhibitors are known to be effective antihypertensive agents in a variety of animal models and are useful clinically, for example, in many human patients with malignant renovascular and essential hypertension.

## EXAMPLES AND PREPARATIONS

The following Examples are provided for illustration of this invention and the best mode of practicing it, and should not be construed to limit it.

### PREPARATION 1

This preparation shows the synthesis of Benzyl -$N^{\alpha}$-t-butyloxycarbonyl-$N^G$,$N^{G'}$-diethyl-L-homoarginyl-L-prolinate.

760 mg (2 mmol) of $N^2$-t-butyloxycarbonyl-$N^G$,$N^{G'}$-diethyl-L-homoarginine hydrochloride was dissolved in 5 ml of DMF. 450 mg (2.2 mmol) of dicyclohexylcarbodiimide and 299 mg (2.2 mmol) of 1 hydroxybenzotriazole (HOBT) was added to the DMF solution. 531 g (2.2 mmol) of benzyl prolinate hydrochloride was added to the solution. The pH was adjusted to between 8 and 9 with N-methylmorpholine. The reaction mixture was stirred at room temperature overnight and filtered. The filtrate was concentrated to dryness. The residue was partitioned between ethyl acetate and water. The water layer was concentrated to dryness and passed through silica gel column using a gradient from dichloromethane/methanol (9:1) to dichloromethane/methanol (4:1) as the eluent. The concentrated fractions gave the product as a white foam $[\alpha]_D^{25}$ -52.9°.

### PREPARATION 2

This preparation shows the synthesis of Benzyl N-[$N^{\alpha}$-(1-ethoxycarbonyl-3-oxo-3-phenylpropyl)-L-($N^G$,$N^{G'}$-diethylhomoarginyl)]-L-prolinate.

1.3 grams (2.2 mm) of benzyl N-t-butyloxycarbonyl-$N^G$,$N^{G'}$-diethyl-L-homoarginyl-L-prolinate was deprotected using 3 ml of saturated hydrochloric acid in ethyl acetate. The deprotected peptide was titrated with diethylether, the ether was decanted and the residue was dried to give 1.1 grams of oil. The oil was dissolved in 5 ml of absolute ethyl alcohol and the pH was adjusted to between 8 and 9 with triethylamine. 0.53 grams (2.59 mmol) of ethyl 3-benzoyl-acrylate was added. The reaction mixture was allowed to stir at room temperature for 2 hours and stripped to dryness. The residue was partitioned between water and ethyl acetate. The water layer was concentrated to dryness and passed through a silica gel column and eluted with dichloromethane methanol 4:1. The appropriate fractions were concentrated to give the product as a white foam.

### PREPARATION 3

13.17 g of benzyl 1,2,3,4-tetrahydroisoquinoline-3-(S)-carboxylate tosylate (N. Yoneda, et al, Chem.Pharm.Bull., 31, 312 (1983)) was suspended in 30mls $CHCl_3$. N-methylmorpholine was added until the pH was 7. 6.8 g of dicyclohexylcarbodiimide and 3.17 g of pyruvic acid was added at -5° C. The reaction mixture was stirred at -50° C for one hour and 0° C for 48 hours. The reaction mixture was filtered,and the filtrate was concentrated to dryness to give a semi-solid. The solid was purified by passing through a silica gel column and eluting with ethylacetate: hexane (1:1) to give benzyl N-pyruvoyl-(3)-1,2,3,4-tetrahydroisoquinoline-S-carboxylate, a yellow oil. $[\alpha]_D^{25}$ -10.3°.

Example 1: Preparation of N,N′-diethylformamidinesulfonic acid.

A. 1,3-diethyl-2-thiourea (85 g, 643 mmol) and sodium molybdate dihydrate (415 mg, 1.7 mmol) were dissolved in water (850 mL), and the resulting solution cooled to 0 - 5° C. Hydrogen peroxide (15%, 410 mL, 1808 mmol) was added dropwise to the solution with stirring over 3 hours, while keeping the temperature below 10° C. After the addition was complete, the solution was stirred at that temperature for one hour, then allowed to warm to room temperature with stirring, and stirred at room temperature for 20 hours. The reaction mixture was then cooled below 10° C and the excess oxidant was destroyed by the addition of solid sodium bisulfite (the addition being ended when the original yellowish color of the solution disappeared). The solution of N,N'-diethylformamidinesulfonic acid was then filtered and used without further treatment in the preparation of a guanidinoamino acid (see Example 2 below). Alternatively, the solution was lyophilized to remove water, and the lyophilized product dissolved in methanol and precipitated with diethyl ether to provide the solid N,N'-diethylformamidinesulfonic acid.

B. Substituting for 1,3-diethyl-2-thioruea, in the procedure of part A of this Example,
1,3-dimethyl-2-thiourea,
1,3-bis(2,2,2-trifluoroethyl)-2-thiourea, and
1,3-di-n-propyl-2-thiourea,
one obtains, respectively,
N,N'-dimethylformamidinesulfonic acid,
N,N'-bis(2,2,2-trifluoroethyl)formamidinesulfonic acid, or
N,N'-di-n-propylformamidinesulfonic acid.

C. Similarly, substituting 1-phenyl-2-thiourea for 1,3-diethyl-2-thiourea, and using a similar procedure to that of Part A of this Example (replacing water as a solvent with methanol/water), there was obtained N-phenylformamidinesulfonic acid.

Example 2: Preparation of Nα-t-butoxycarbonyl-$N^G$,$N^{G'}$-diethyl-(R)-homoarginine (BOC-Deh).

A. To (R)-lysine hydrochloride (104 g, 569 mmol) was added sufficient 10% aqueous sodium hydroxide to give a solution of pH 10.65. The solution was cooled in an ice bath, and the solution of N,N'-diethylformamidinesulfonic acid prepared in Part A of Example 1 added dropwise, with vigorous stirring, over 2 hours. During the addition, the pH was maintained at 10.65±0.05 by the addition of concentrated (20%) aqueous sodium hydroxide solution. After the addition was complete, the solution was allowed to warm to room temperature with stirring, and stirred at room temperature overnight. At this point, $N^G$,$N^{G'}$-diethyl-(R)-homoarginine may be isolated from the solution if desired. However, since the acid is conventionally used synthetically as the Nα-t-BOC derivative, it was convenient to derivatize it without isolation.

The reaction mixture was cooled to 0° C, dioxane (1 L) added, and di-t-butyl dicarbonate (200 g, 915 mmol) in dioxane (200 mL) added dropwise, with stirring. The mixture was stirred at room temperature for three hours, maintaining the pH at 10.0±0.3 by the addition of 10% aqueous sodium hydroxide solution. The solution was then filtered, and the filter cake washed with methanol The washings were combined with the filtrate, and the resulting solution reduced to half its volume. The solution was then extracted with ethyl acetate (2 x 100 mL), acidified to pH 6.4 with 10% aqueous hydrochloric acid, and again extracted with ethyl acetate (2 x 100 mL). The resulting aqueous solution was then evaporated to dryness, and the residue dissolved in a minimum quantity of ethanol at 50° C and filtered. Silica gel (600 g) was added to the solution, which was then evaporated to dryness. The solid was added to a 2 Kg silica gel column packed in acetonitrile, and eluted with acetonitrile (2 L), acetonitrile/water 95:5 (8 L), acetonitrile/water 90:10 (8 L), acetonitrile/water 85:15 (8L), and acetonitrile/water 80:20 (16 L). The fractions containing pure BOC-Deh (examined by tlc on silica gel, eluted with acetonitrile/water 80:20) were combined, evaporated to dryness, redissolved in warm ethanol, filtered, evaporated, triturated with ether, and dried under vacuum. 160 g of pure BOC-Deh was obtained m.p. 89-91° c.

B. Similarly, following the procedure of Part A of this Example and substituting (S)-ornithine hydrochloride for (R)-lysine hydrochloride, there was obtained Nα-t-butoxycarbonyl-$N^G$,$N^{G'}$-diethyl-(S)-arginine IR: 3200, 1690 $cm^{-1}$.

C. Similarly, following the procedure of Part A of this Example and substituting a solution of N-phenylformamidinesulfonic acid for the solution of N,N'-diethylformamidinesulfonic acid, there was obtained Nα-t-butoxycarbonyl-$N^G$-phenyl-(R)-homoarginine MS ($MH^+$) 365.

Example 3: Preparation of N-[$N^\alpha$-(1-ethoxycarbonyl-3-phenylpropyl)-L-($N^G$,$N^{G'}$-diethylhomoarginyl]-L-proline.

400 mg of benzyl N-[$N^\alpha$-(1-ethoxycarbonyl-3-oxo-3-phenylpropyl)-L-($N^G$,$N^{G'}$-diethylhomoarginyl)]-L-prolinate was dissolved in 15 ml of acetic acid with 1.6% concentrated sulfuric acid. 100 mg of 10% palladium

on carbon was added. The reaction mixture was hydrogenated at room temperature under atmospheric pressure for one and one-half hours before being filtered through Celite and neutralized to pH 3 to 4 with dilute ammonium hydroxide at 0 °C. The filtrate was passed through an Amberlite XAD column by elution with a water to ethyl alcohol gradient. The concentration of the appropriate fractions gave about 200 mg of oil. The oil was further purified by preparation reversed phase HPLC on a 2.5x100cm column of Lichroprep RP-18(20-40μ diameter, E. Merck) using an 80% $CH_3CN$ : 20% $H_2O$ (0.03M in $NH_4OAc$, pH 7) eluent (17ml/min), to give 80 mg of one isomer and 40 mg of the other isomer. The compounds are isomeric in position one of the phenylpropyl residue and maybe used in either the racemic or resolved forms.

Similarly, by incorporation of a conventional saponification step, the corresponding diacids can be obtained.

Example 4- Preparation of (S)-2-[(S)-N-[(S)-1-carboxy-5-N′,N″-diethylguanidinopentyl]alanyl]-1,2,3,4-tetrahydroisoquinoline-3-carboxylic acid.

A. An aqueous solution containing 11.4 mmol of (S)-2-[(S)-N-[(S)-1-carboxy-5-aminopentyl]alanyl]-1,2,3,4-tetrahydroisoquinoline-3-carboxylic acid (prepared by the methods described in European Patent Publication Numbers 12401, 65301 and 79022, referred to previously) was adjusted to pH 10.3 by the addition of solid sodium hydroxide. N,N′-Diethylformamidinesulfonic acid (1 g, 5.5 mmol) was added in one portion, with stirring, and the pH adjusted to 10.7 with 20% aqueous sodium hydroxide solution. Additional N,N′-diethylformamidinesulfonic acid (4.6 g, 25.5 mmol) was added in small portions over the next 30 hours, and 20% aqueous sodium hydroxide added as needed to maintain pH 10.6 - 10.9. When the starting peptide had been fully consumed (as shown by tlc), the reaction mixture was acidified to pH 4.5 with glacial acetic acid; and the resulting solution applied to a reverse phase preparative HPLC column of Lichoprep C18 and eluted with acetonitrile/aqueous ammonium acetate (0.05 M, pH 4.5) to effect purification. The purified guanidinopeptide (m.p. 140-150 °C) is isolated from the appropriate column fractions (tested by tlc or hplc) by standard techniques well-known to those of ordinary skill in the art. In particular, the previously described interconversion steps (m), (n) or (o) provide for the isolation of the free base of the guanidinopeptide, a salt of the guanidinopeptide or an acid addition salt of the guanidinopeptide.

B. N,N′-diethylformamidinesulfonic acid (4.94 g, 27.4 mmol) was added in one portion to a solution of benzyl (S)-2-[(S)-N-[(S)-1-methoxycarbonyl-5-aminopentyl]alanyl]-1,2,3,4-tetrahydroisoquinoline-3-carboxylate (14.65 g, 30.5 mmol) (prepared by the methods described in European Patent Publication Numbers 12401, 65301 and 79022, referred to previously) in acetonitrile (115 mL), with stirring at room temperature. After stirring at room temperature overnight, the reaction mixture was applied to a silica gel column, and eluted with acetronitrile/acetic acid/water mixtures to effect purification. Purified benzyl (S)-2-[(S)-N-[(S)-1-methoxycarbonyl-5-N′,N″-diethylguanidinopentyl]alanyl]-1,2,3,4-tetrahydroisoquinoline-3-carboxylate was isolated from the appropriate column fractions (tested by tlc) by standard techniques well-known to those of ordinary skill in the art.

Benzyl (S)-2-[(S)-N-[(S)-1-methoxycarbonyl-5-N′,N″-diethyl-guanidinopentyl]alanyl]-1,2,3,4-tetrahydroisoquinoline-3-carboxylate was hydrolyzed in 0.63 N sodium hydroxide for 1 hour and then acidified to about pH 4 with hydrochloric acid. The reaction mixture was passed through an Amberlite XAD-2 resin column, eluted with water and then a gradient of water to ethanol. The crude product obtained was further purified by preparative reversed phase HPLC on a 2.5x100cm column of Lichroprep RP-18(20-40μ diameter, E. Merck) using an 18% $CH_3CN$ : 72% $H_2O$ (0.03M in $NH_4OAc$, pH 7) eluent (17ml/min) to give the title compound; m.p. 140-150 °C. The previously described interconversion steps (m), (n) or (o) provide for the isolation of the free base of the title compound, a salt of the title compound or an acid addition salt of the title compound.

EXAMPLE 5

The following compounds were prepared using the methods identified in the tabulation below with the corresponding physical constants.

($R^5$ = hydroxy, $R^6$ = 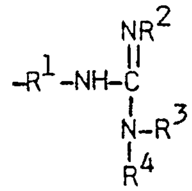 )

| $R^7$ | 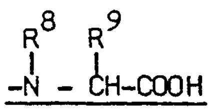 | $R^2$ | $R^3$ | $R^4$ | Method | Physical Constant |
|---|---|---|---|---|---|---|
| $CH_3$ | THIQ | $C_2H_5$ | $C_2H_5$ | H | (d) | m.p. 140-150°C |
| | | | | | (f) | nmr:m/s:[M+H] 476 |
| | | | | | (c) | m.p. 142-152°C |
| | | | | | (e) | nmr confirmed |
| | | | | | (h) | nmr confirmed |
| $CH_3$ | THIQ | $CH_3$ | n-$C_4H_9$ | H | (d) | |
| | | | | | (f) | |
| | | | | | (c) | |
| | | | | | (e) | |
| | | | | | (h) | |
| | | | | | (g) | |
| $CH_3$ | THIQ | $CF_3CH_2$ | $CF_3CH_2$ | H | (d) | m/s[$MH^+H_2O$] 565 |
| | | | | | (g) | nmr confirmed |
| $C_2H_5$ | THIQ | $C_2H_5$ | $C_2H_5$ | H | (a) | $[M+H]^+$ 490 |
| | | | | | (g) | $[M+H]^+$ 490 |
| $CH_3$ | 7-$CH_3$-THIQ | $C_2H_5$ | $C_2H_5$ | H | (d) | |
| | | | | | (g) | |
| $CH_3$ | 7-Cl-THIQ | $C_2H_5$ | $C_2H_5$ | H | (j) | |
| | | | | | (g) | |
| $CH_3$ | prolin-N-yl | $C_2H_5$ | $C_2H_5$ | H | (d) | |
| $C_2H_5$ | prolin-N-yl | $C_2H_5$ | $C_2H_5$ | H | (d) | $[M+H]^+$ 448 |
| | | | | | (f) | nmr confirmed |
| $CH_3$ | prolin-N-yl | $CF_3CH_2$ | $CF_3CH_2$ | H | (a) | $[M+H]^+$ 522 |
| | | | | | (f) | nmr confirmed |
| $CH_3$ | 2-carboxy-indol-N-yl | $C_2H_5$ | $C_2H_5$ | H | (d) | $[M+H]^+$ 444 |
| | | | | | (c) | |

| $R^7$ | $R^8$ $R^9$ -N - CH-COOH | $R^2$ | $R^3$ | $R^4$ | Method | Physical Constant |
|---|---|---|---|---|---|---|
| $CH_3$ | THIQ | $CH_3$ | $CH_3$ | H | (d) | |
| | | | | | (f) | |
| | | | | | (c) | |
| | | | | | (e) | |
| | | | | | (h) | |

**Claims**

**1.** A method for preparing a compound of the formula:

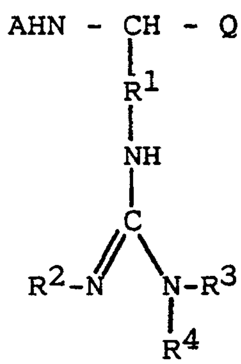

(III)

or the pharmaceutically acceptable, non-toxic salts thereof, in which

A is H, R (wherein R is: optionally substituted lower alkyl, namely branched or unbranched alkyl of from 1 to 6 carbon atoms wherein one or more hydrogen atoms is optionally substituted by lower alkyl of from 1 to 3 carbon atoms, lower alkoxy of from 1 to 3 carbon atoms, halogen or trifluoromethyl; phenyl wherein one or more hydrogen atoms is optionally substituted by lower alkyl of from 1 to 3 carbon atoms, lower alkoxy of from 1 to 3 carbon atoms, halogen or trifluoromethyl; or aralkyl selected from phenyl-substituted lower alkyl of from 1 to 3 carbon atoms wherein one or more hydrogen atoms is optionally substituted by lower alkyl of from 1 to 3 carbon atoms, lower alkoxy of from 1 to 3 carbon atoms, halogen or trifluoromethyl), R-O-CO-, peptidyl residue, or (peptidyl residue)-CO-(lower alkyl as defined above);

Q is a carboxylate-related group;

$R^1$ is optionally substituted lower alkylene namely an unbranched saturated hydrocarbon chain containing from 2 to 6 carbon atoms wherein one or more hydrogen atoms is optionally substituted by lower alkyl of from 1 to 3 carbon atoms, lower alkoxy of from 1 to 3 carbon atoms, halogen or trifluoromethyl; and

$R^2$, $R^3$, and $R^4$ are each independently H or R (as defined above), with the proviso that at least one of $R^2$, $R^3$, and $R^4$ is not hydrogen,

which method comprises

(a) alkylating a compound of the formula:

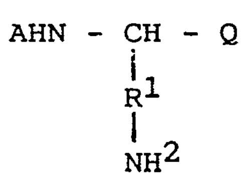

(I)

or a functional derivative thereof in which

A, Q, and $R^1$ are as previously defined with a formamidinesulfonic acid of the formula:

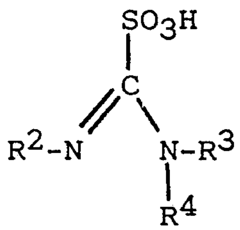

(II)

in which

$R^2$, $R^3$, and $R^4$ are as previously defined and optionally;

(b) converting an acid addition salt of the compound of formula (III) with base to the compound of formula (III) or a salt thereof with said base; or

(c) converting a salt of the compound of formula (III) (derived from a base) with acid to the free compound of formula (III) or an acid addition salt thereof; or

(d) converting a salt of the compound of formula (III), preferably a soluble salt, to another salt of the compound of formula (III), preferably less soluble than said soluble salt.

**2.** The method of Claim 1 wherein A is H or R-O-CO- (wherein R is as defined in Claim 1).

**3.** The method of Claim 2 wherein A is H.

**4.** The method of Claim 2 wherein A is t-butoxycarbonyl.

**5.** The method of any one of Claims 1-4 wherein Q is -COOH or -COOR (wherein R is as defined in Claim 1).

**6.** The method of any one of the preceding Claims wherein $R^1$ is $-(CH_2)_n-$ and n is an integer of 3 to 5.

**7.** The method of any one of the preceding Claims wherein each of $R^2$ and $R^3$ are independently optionally substituted lower alkyl (as defined in Claim 1) and $R^4$ is hydrogen.

**8.** The method of any one of the preceding Claims wherein each of $R^2$ and $R^3$ is optionally substituted ethyl wherein one or more hydrogen atoms is optionally substituted by lower alkyl of from 1 to 3 carbon atoms, lower alkoxy of from 1 to 3 carbon atoms, halogen or trifluoromethyl.

**9.** The method of any one of Claims 1-6 wherein $R^2$ is optionally substituted phenyl wherein one or more hydrogen atoms is optionally substituted by lower alkyl of from 1 to 3 carbon atoms, lower alkoxy of from 1 to 3 carbon atoms, halogen or trifluoromethyl and $R^3$ and $R^4$ are each hydrogen.

**10.** The method of Claim 9 wherein $R^2$ is phenyl.

**11.** The method of Claim 1 wherein the compound of formula I is lysine.

**12.** The method of Claim 11 wherein the formamidinesulfonic acid of formula II is N,N'-diethylformamidinesulfonic acid.

**13.** The method of Claim 1 wherein A is (peptidyl residue)-CO-(lower alkyl)-, Q is -COOH or -COOR, and $R^1$ is $-(CH_2)_n-$ where n is an integer of 3 to 5.

**14.** The method of Claim 13 wherein each of $R^2$ and $R^3$ are independently optionally substituted lower alkyl and $R^4$ is hydrogen.

**15.** The method of Claim 14 wherein each of $R^2$ and $R^3$ is ethyl.

**16.** The method of any one of Claims 13-15 wherein the compound of formula I is (S)-2-[(S)-N-[(S)-1-carboxy-5-aminopentyl]-alanyl]-1,2,3,4-tetrahydroisoquinoline-3-carboxylic acid or a salt thereof or a mono- or di-ester thereof.

**17.** The method of Claim 16 when dependent on Claim 15 wherein (S)-2-[(S)-N-[(S)-1-carboxy-5-N',N"diethylguanidinopentyl]alanyl]-1,2,3,4-tetrahydro-isoquinoline-3-carboxylic acid or a pharmaceutically acceptable, non-toxic salt thereof is prepared.

**18.** The method of any one of the preceding Claims which comprises the preparation of a $N^G,N^{G'}$-dialkylguanidino peptide from a compound of the formula III.

**19.** The method of any one of the preceding claims which comprises the preparation of ACE inhibitors.

**20.** The method of any one of the preceding claims for the preparation of angiotensin converting enzyme inhibitors of the formula A

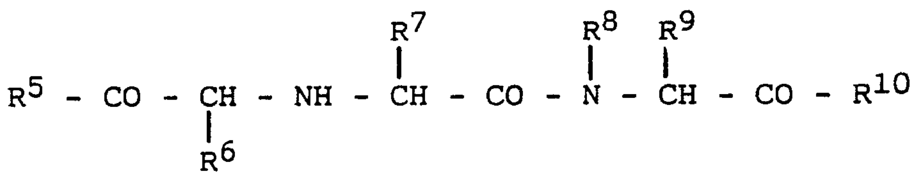
(A)

wherein $R^5$ represents hydroxy, lower alkoxy ( - OR where R is lower alkyl as defined below), benzyloxy or amino optionally substituted with one or two lower alkyl groups, namely branched or unbranched saturated hydrocarbon chains containing from 1 to 6 carbon atoms;

R6 and $R^7$ represent

(1) hydrogen or preferably lower alkyl (as defined above) (optionally omega-substituted with phenyl or naphthyl, less preferred omega-substituted with di(lower)alkylguanidino), or

(2)
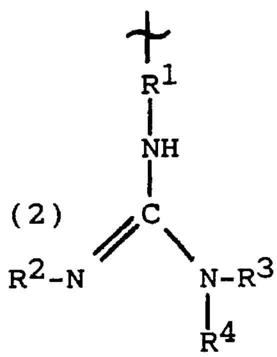

wherein $R^1$ is optionally substituted lower alkylene (as defined in claim 1) and $R^2$, $R^3$ and $R^4$ are each selected from H and R (wherein R is optionally substituted lower alkyl, phenyl, and aralkyl, all as defined in claim 1), with the proviso that at least one of $R^2$, $R^3$, and $R^4$ is not hydrogen; n is an integer from 3 to 5, preferably 4; and one of $R^6$ and $R^7$ being (1), the other being (2); and the group -

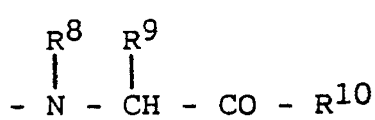

is an amino acid residue selected from the group of imino acid residues, preferably cyclic imino acid residues in which the subgroup

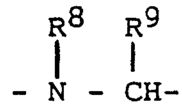

forms a heterocyclic radical containing one nitrogen atom and up to 9 ring carbon atoms, in particular said cyclic imino acid residue is or is derived from proline, hydroxyproline or proline analogs;

$R^{10}$ is hydroxy, lower alkoxy (as defined above), benzyloxy or amino optionally substituted with one or two lower alkyl groups (as defined above); and the pharmaceutically acceptable, non-toxic salts thereof, which further comprises:

(a) condensing an amino acid derivative of the formula

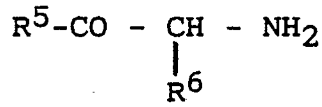

wherein $R^6$ is the group (2) above,
with an α-ketoacid derivative of the formula

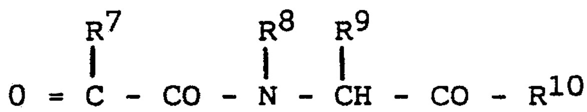

under reductive conditions wherein $R^5$, $R^7$, $R^8$, $R^9$ and $R^{10}$ are defined above; or
(b) condensing an α-ketoacid derivative of the formula

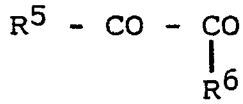

with a peptide of the formula

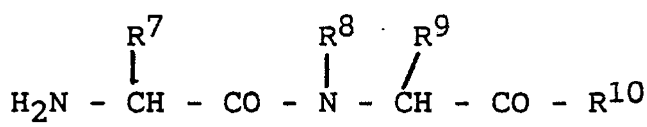

wherein $R^7$ is the group (2) above,
under reductive conditions wherein $R^5$, $R^6$, $R^8$, $R^9$ and $R^{10}$ are defined above; or
(c) condensing an amino acid derivative of the formula

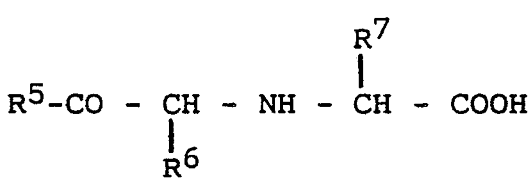

with proline or a proline analog derivative of the formula

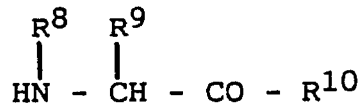

wherein $R^5$, $R^6$, $R^7$, $R^8$, $R^9$ and $R^{10}$ are defined above; or
(d) alkylating a peptide derivative of the formula

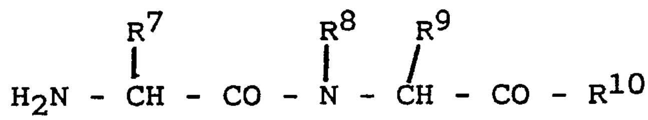

wherein $R^7$ is the group (2) above, with an alkylating agent of the formula

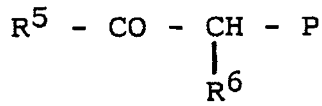

wherein P is halo selected from the group of chloro, bromo or iodo, or is a sulfonyloxy group and $R^5$, $R^6$, $R^8$, $R^9$ and $R^{10}$ are defined above; or
(e) alkylating an amino acid derivative of the formula

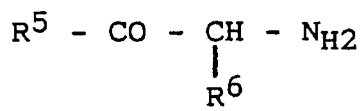

wherein $R^6$ is the group (2) above, with an alkylating agent of the formula

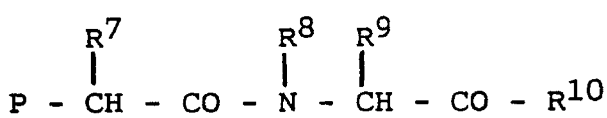

wherein P has the meanings of step (e) and $R^5$, $R^7$, $R^8$, $R^9$ and $R^{10}$ are defined above; or
(f) hydrolyzing a compound of the formula

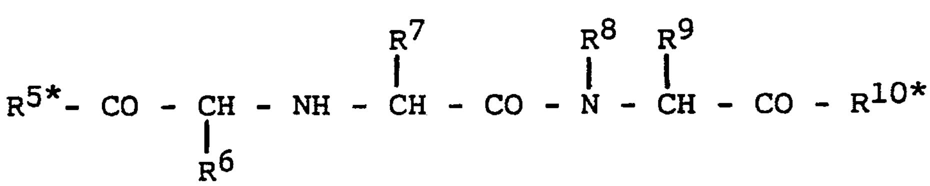

wherein $R^{5*}$ and $R^{10*}$ are hydroxy, lower alkoxy (as defined above) or benzyloxy and at least one of $R^{5*}$ and $R^{10*}$ is lower alkoxy or benzyloxy to a compound of formula A wherein $R^5$ and $R^{10}$ are hydroxy; or
(g) hydrogenolyzing (reductively cleaving) a compound of the formula

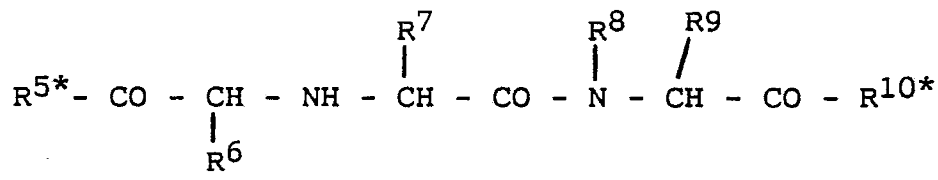

wherein $R^{5*}$ and $R^{10*}$ are hydroxy, lower alkoxy (as defined above) or benzyloxy and at least one of $R^{5*}$ and $R^{10*}$ is benzyloxy to a compound of formula A wherein at least one of $R^5$ and $R^{10}$ is hydroxy; or

(h) reacting a compound of formula A with a base to form a salt of the compound of formula A; or

(i) reacting a compound of formula A with an acid to form an acid addition salt of the compound of formula A; or

(j) esterifying a compound of the formula

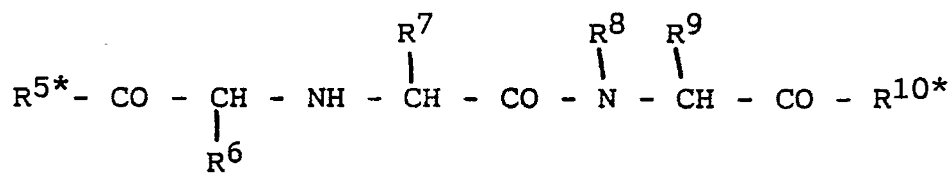

wherein at least one of $R^{5*}$ and $R^{10*}$ is hydroxy and the other is hydroxy, lower alkoxy or benzyloxy with a lower alkanol or benzyl alcohol; or

(k) acylating ammonia, a lower alkyl amine or a di(lower)alkyl amine with a dipeptide derivate of the formula

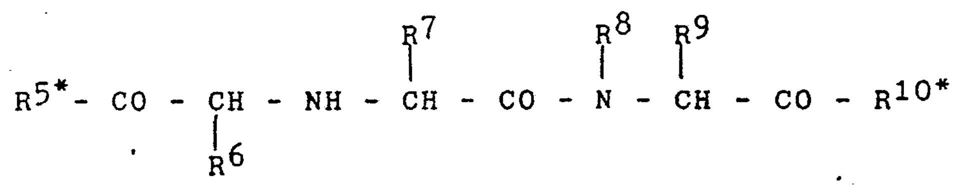

wherein $R^{5*}$ and $R^{10*}$ are hydroxy, chloro, bromo, lower alkoxy, benzyloxy, a lower alkanoyloxy group, or amino optionally substituted with one or two lower alkyl groups, and at least one of $R^{5*}$ and $R^{10*}$ is hydroxy, chloro, bromo, lower alkoxy, benzyloxy, or a lower alkanoyloxy group; and

(l) converting an acid addition salt of the peptide of formula A with base to the peptide of formula A or a salt thereof with said base; or

(m) converting a salt of a peptide of formula A (derived from a base) with acid to the free peptide of formula A or an acid addition salt thereof; or

(n) converting a salt of the peptide of formula A, preferably a soluble salt to another salt of the peptide of formula A, preferably less soluble than said soluble salt.

## Revendications

**1.** Procédé de préparation d'un composé de formule :

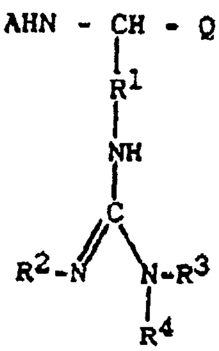

(III)

ou de ses sels non toxiques acceptables du point de vue pharmaceutique, formule dans laquelle

A représente H, un groupe R (R représentant : un groupe alkyle inférieur facultativement substitué, à savoir un groupe alkyle ramifié ou non ramifié de 1 à 6 atomes de carbone dont un ou plusieurs atomes d'hydrogène sont facultativement substitués par un radical alkyle inférieur ayant 1 à 3 atomes de carbone, alkoxy inférieur ayant 1 à 3 atomes de carbone, halogéno ou trifluorométhyle ; un groupe phényle dont un ou plusieurs atomes d'hydrogène sont facultativement substitués par un radical alkyle inférieur ayant 1 à 3 atomes de carbone, alkoxy inférieur ayant 1 à 3 atomes de carbone, halogéno ou trifluorométhyle ; ou un groupe aralkyle choisi entre un groupe alkyle inférieur à substituant phényle ayant 1 à 3 atomes de carbone, dont un ou plusieurs atomes d'hydrogène sont facultativement substitués par un radical alkyle inférieur ayant 1 à 3 atomes de carbone, alkoxy inférieur ayant 1 à 3 atomes de carbone, halogéno ou trifluorométhyle), un groupe R-O-CO-, un résidu peptidyle ou un groupement (résidu peptidyle)-CO-(alkyle inférieur tel que défini ci-dessus) ;

Q est un groupe en rapport avec un carboxylate ;

$R^1$ est un groupe alkylène inférieur facultativement substitué, à savoir une chaîne hydrocarbonée saturée non ramifiée contenant 2 à 6 atomes de carbone dont un ou plusieurs atomes d'hydrogène sont facultativement substitués par un radical alkyle inférieur ayant 1 à 3 atomes de carbone, alkoxy inférieur ayant 1 à 3 atomes de carbone, halogéno ou trifluorométhyle ; et

$R^2$, $R^3$ et $R^4$ représentent chacun, indépendamment, H ou R (tel que défini ci-dessus), sous réserve qu'au moins l'un de $R^2$, $R^3$ et $R^4$ ne soit pas l'hydrogène,

procédé qui consiste

(a) à alkyler un composé de formule :

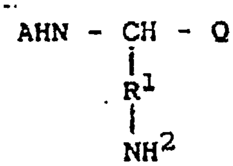

(I)

ou un dérivé fonctionnel de ce composé, formule dans laquelle

A, Q et $R^1$ sont tels que définis ci-dessus, avec un acide formamidinesulfonique de formule :

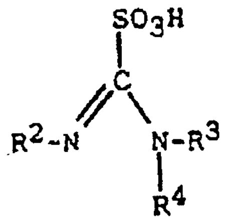

(II)

dans laquelle

$R^2$, $R^3$ et $R^4$ sont tels que définis ci-dessus et, à titre facultatif :

(b) à convertir un sel d'addition d'acide du composé de formule (III) avec une base en le composé de formule (III) ou un sel de ce composé avec ladite base ; ou

(c) à convertir un sel du composé de formule (III) (dérivé d'une base) avec un acide en le composé libre de formule (III) ou un sel d'addition d'acide de ce composé ; ou

(d) à convertir un sel du composé de formule (III), de préférence un sel soluble, en un autre sel du composé de formule (III), de préférence moins soluble que le sel soluble en question.

**2.** Procédé suivant la revendication 1, dans lequel A représente H ou R-O-CO- (où R est tel que défini dans la revendication 1).

**3.** Procédé suivant la revendication 2, dans lequel A représente H.

**4.** Procédé suivant la revendication 2, dans lequel A est un groupe tertio-butoxycarbonyle.

**5.** Procédé suivant l'une quelconque des revendications 1 à 4, dans lequel Q est un groupe -COOH ou -COOR (où R est tel que défini dans la revendication 1).

**6.** Procédé suivant l'une quelconque des revendications précédentes, dans lequel $R^1$ est un groupe $-(CH_2)_n-$ et n est un nombre entier de 3 à 5.

**7.** Procédé suivant l'une quelconque des revendications précédentes, dans lequel chacun de $R^2$ et $R^3$ représente, indépendamment, un groupe alkyle inférieur facultativement substitué (tel que défini dans la revendication 1) et $R^4$ est l'hydrogène.

**8.** Procédé suivant l'une quelconque des revendications précédentes, dans lequel chacun de $R^2$ et $R^3$ est un groupe éthyle facultativement substitué dont un ou plusieurs atomes d'hydrogène sont facultativement substitués par un radical alkyle inférieur ayant 1 à 3 atomes de carbone, alkoxy inférieur ayant 1 à 3 atomes de carbone, halogéno ou trifluorométhyle.

**9.** Procédé suivant l'une quelconque des revendications 1 à 6, dans lequel $R^2$ est un groupe phényle éventuellement substitué dont un ou plusieurs atomes d'hydrogène sont facultativement substitués par un radical alkyle inférieur ayant 1 à 3 atomes de carbone, alkoxy inférieur ayant 1 à 3 atomes de carbone, halogéno ou trifluorométhyle et $R^3$ et $R^4$ représentent chacun de l'hydrogène.

**10.** Procédé suivant la revendication 9, dans lequel $R^2$ est le groupe phényle.

**11.** Procédé suivant la revendication 1, dans lequel le composé de formule I est la lysine.

**12.** Procédé suivant la revendication 11, dans lequel l'acide formamidinesulfonique de formule II est l'acide N,N'-diéthylformamidinesulfonique.

**13.** Procédé suivant la revendication 1, dans lequel A est un groupement (résidu peptidyle)-CO-(alkyle inférieur)-, Q est un groupe -COOH ou -COOR et $R^1$ est un groupe $-(CH_2)n-$ dans lequel $\underline{n}$ est un nombre entier de 3 à 5.

**14.** Procédé suivant la revendication 13, dans lequel chacun de $R^2$ et $R^3$ représente, indépendamment, un groupe alkyle inférieur facultativement substitué et $R^4$ est l'hydrogène.

**15.** Procédé suivant la revendication 14, dans lequel chacun de $R^2$ et $R^3$ est un groupe éthyle.

**16.** Procédé suivant l'une quelconque des revendications 13 à 15, dans lequel le composé de formule I est l'acide (S)-2-[(S)-N-[(S)-1-carboxy-5-aminopentyl]-alanyl]-1,2,3,4-tétrahydro-isoquinoléine-3-carboxylique ou un sel ou un mono-ester ou un diester de cet acide.

**17.** Procédé suivant la revendication 16 subordonnée à la revendication 15, dans lequel on prépare l'acide (S)-2-[(S)-N-[(S)-1-carboxy-5-N',N''-diéthylguanidinopentyl]alanyl]-1,2,3,4-tétrahydro-isoquinoléine-3-carboxylique ou un sel non toxique pharmaceutiquement acceptable de cet acide.

**18.** Procédé suivant l'une quelconque des revendications précédentes, qui comprend la préparation d'un $N^G,N^{G'}$-dialkylguanidinopeptide à partir d'un composé de formule III.

**19.** Procédé suivant l'une quelconque des revendications précédentes, qui comprend la préparation

d'inhibiteurs de l'enzyme transformant l'angiotensine.

**20.** Procédé suivant l'une quelconque des revendications précédentes, destiné à la préparation d'inhibiteurs de l'enzyme transformant l'angiotensine, de formule A

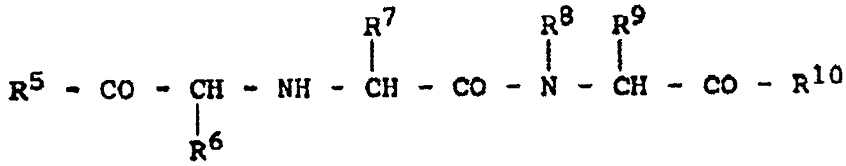 (A)

dans laquelle $R^5$ représente un groupe hydroxy, alkoxy inférieur (-OR où R est un radical alkyle inférieur tel que défini ci-dessous), benzyloxy ou amino facultativement substitué avec un ou deux groupes alkyle inférieur, à savoir des chaînes hydrocarbonées saturées ramifiées ou non ramifiées contenant 1 à 6 atomes de carbone ;

$R^6$ et $R^7$ représentent

(1) l'hydrogène ou de préférence un groupe alkyle inférieur (tel que défini ci-dessus) (le cas échéant oméga-substitué avec un radical phényle ou naphtyle, moins préférentiellement oméga-substitué avec un groupe di(alkyle inférieur)guanidino), ou

(2)

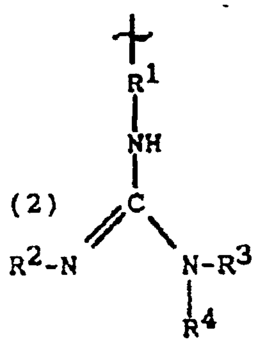

où $R^1$ est un groupe alkylène inférieur facultativement substitué (tel que défini dans la revendication 1) et $R^2$, $R^3$ et $R^4$ sont choisis chacun entre H et R (où R représente un groupe alkyle inférieur, un groupe phényle et un groupe aralkyle facultativement substitués, tous tels que définis dans la revendication 1), sous réserve qu'au moins l'un de $R^2$, $R^3$ et $R^4$ ne représente pas l'hydrogène ; n est un nombre entier de 3 à 5, de préférence 4 ; et l'un de $R^6$ et $R^7$ correspondant à l'option (1), l'autre correspondant à l'option (2) ; et le groupe

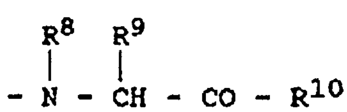

est un résidu d'amino-acide choisi dans le groupe des résidus d'imino-acides, de préférence des résidus d'iminoacides cycliques, dans lequel le sous-groupe

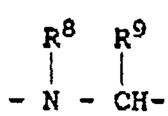

forme un radical hétérocyclique contenant un atome d'azote et jusqu'à 9 atomes cycliques de carbone, en particulier ledit résidu d'imino-acide cyclique étant la proline, l'hydroxyproline ou les analogues de

proline ou en étant dérivé ;

$R^{10}$ est un groupe hydroxy, alkoxy inférieur (tel que défini ci-dessus), benzyloxy ou amino facultativement substitué avec un ou deux radicaux alkyle inférieurs (tels que définis ci-dessus) ; et de leurs sels non toxiques pharmaceutiquement acceptables, qui consiste en outre :

(a) à condenser un dérivé d'amino-acide de formule :

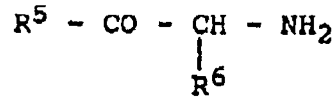

dans laquelle $R^6$ est le groupe (2) ci-dessus,

avec un dérivé d'α-céto-acide de formule

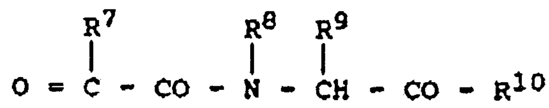

dans des conditions de réduction, formules dans lesquelles $R^5$, $R^7$, $R^8$, $R^9$ et $R^{10}$ ont les définitions données ci-dessus ; ou bien

(b) à condenser un dérivé d'α-céto-acide de formule

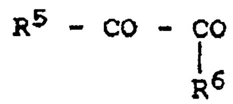

avec un peptide de formule

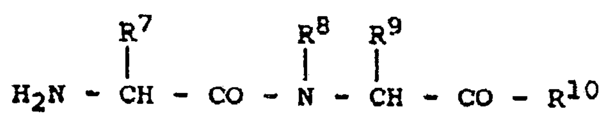

dans laquelle $R^7$ est le groupe (2) ci-dessus,

dans des conditions de réduction, formules dans lesquelles $R^5$, $R^6$, $R^8$, $R^9$ et $R^{10}$ sont tels que définis ci-dessus ; ou bien

(c) à condenser un dérivé d'amino-acide de formule

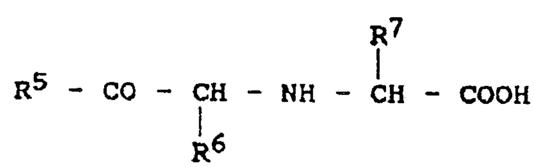

avec la proline ou un dérivé analogue de proline de formule

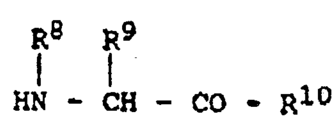

dans laquelle $R^5$, $R^6$, $R^7$, $R^8$, $R^9$ et $R^{10}$ sont tels que définis ci-dessus ; ou bien

(d) à alkyler un dérivé peptidique de formule

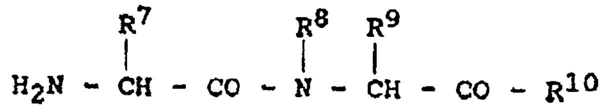

dans laquelle $R^7$ est le groupe (2) ci-dessus,

avec un agent alkylant de formule

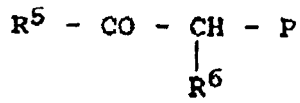

dans laquelle P est un radical halogéno choisi dans le groupe des radicaux chloro, bromo ou iodo, ou est un groupe sulfonyloxy et $R^5$, $R^6$, $R^8$, $R^9$ et $R^{10}$ sont tels que définis ci-dessus ; ou bien

(e) à alkyler un dérivé d'amino-acide de formule

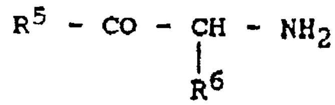

dans laquelle $R^6$ est le groupe (2) ci-dessus,

avec un agent alkylant de formule

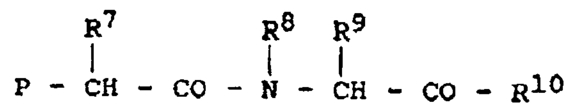

dans laquelle P a les définitions de l'étape (e) et $R^5$, $R^7$, $R^8$, $R^9$ et $R^{10}$ sont tels que définis ci-dessus ; ou bien

(f) à hydrolyser un composé de formule

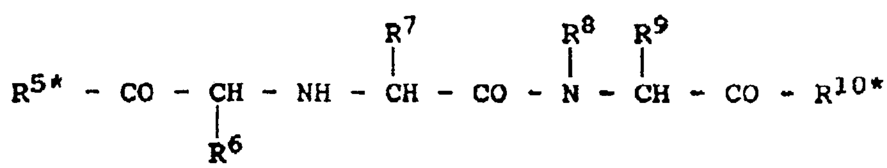

dans laquelle $R^{5*}$ et $R^{10*}$ représentent un groupe hydroxy, alkoxy inférieur (tel que défini ci-dessus) ou benzyloxy et l'un au moins de $R^{5*}$ et $R^{10*}$ est un groupe alkoxy inférieur ou benzyloxy, en un coposé de formule A dans laquelle $R^5$ et $R^{10}$ sont des groupes hydroxy ; ou bien

(g) à hydrogénolyser (clivage par réduction) un composé de formule

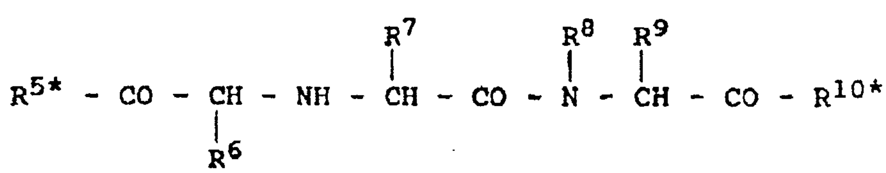

dans laquelle $R^{5*}$ et $R^{10*}$ sont des groupes hydroxy, alkoxy inférieur (tel que défini ci-dessus) ou benzyloxy et l'au moins de $R^{5*}$ et $R^{10*}$ est un groupe benzyloxy, en un composé de formule A dans laquelle l'un au moins de $R^5$ et $R^{10}$ est un groupe hydroxy ; ou bien

(h) à faire réagir un composé de formule A avec une base pour former un sel du composé de formule A ; ou bien

(i) à faire réagir un composé de formule A avec un acide pour former un sel d'addition d'acide du composé de formule A ; ou bien

(j) à estérifier un composé de formule

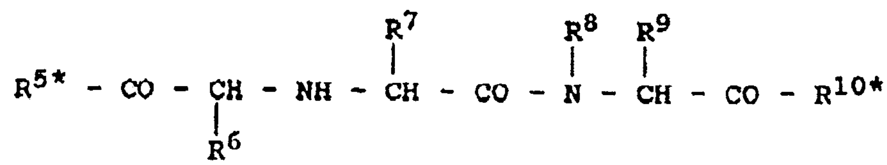

dans laquelle l'un au moins de $R^{5*}$ et $R^{10*}$ est un groupe hydroxy et l'autre est un groupe hydroxy, alkoxy inférieur ou benzyloxy, avec un alcanol inférieur ou l'alcool benzylique ; ou bien

(k) à acyler de l'ammoniac, une alkylamine inférieure ou une di(alkyle inférieur)amine avec un dérivé dipeptidique de formule

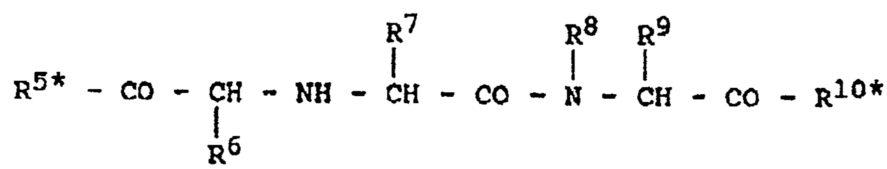

dans laquelle $R^{5*}$ et $R^{10*}$ sont des radicaux hydroxy, chloro, bromo, alkoxy inférieur, benzyloxy, un groupe alcanoyloxy inférieur ou un groupe amino facultativement substitué avec un ou deux groupes alkyle inférieurs, et l'un au moins de $R^{5*}$ et $R^{10*}$ est un radical hydroxy, chloro, bromo, alkoxy inférieur, benzyloxy ou un groupe alcanoyloxy inférieur ; et

(l) à convertir un sel d'addition d'acide du peptide de formule A avec une base en le peptide de formule A ou en un sel de ce peptide avec ladite base ; ou bien

(m) à convertir un sel d'un peptide de formule A (dérivé d'une base) avec un acide en le peptide libre de formule A ou en un sel d'addition d'acide de ce peptide ; ou bien

(n) à convertir un sel du peptide de formule A, de préférence un sel soluble, en un autre sel du peptide de formule A, de préférence moins soluble que le sel soluble en question.

## Patentansprüche

1. Verfahren zur Herstellung einer Verbindung der Formel:

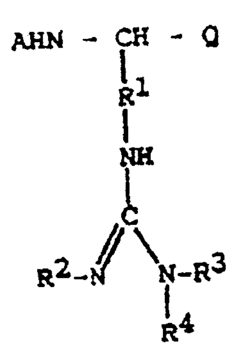

(III)

oder der pharmazeutisch annehmbaren, nicht-toxischen Salze derselben, in welcher

A für H, R (worin R bedeutet: gegebenenfalls substituiertes Niederalkyl, nämlich verzweigtes oder unverzweigtes Alkyl mit 1 bis 6 Kohlenstoffatomen, worin ein oder mehrere Wasserstoffatome gegebenenfalls durch Niederalkyl mit 1 bis 3 Kohlenstoffatomen. Niederalkoxy mit 1 bis 3 Kohlenstoffatomen, Halogen oder Trifluormethyl substituiert sind; Phenyl, worin ein oder mehrere Wasserstoffatome gegebenenfalls durch Niederalkyl mit 1 bis 3 Kohlenstoffatomen. Niederalkoxy mit 1 bis 3 Kohlenstoffatomen, Halogen oder Trifluormethyl substituiert sind; oder Aralkyl, ausgewählt aus Phenyl-substituiertem Niederalkyl mit 1 bis 3 Kohlenstoffatomen, worin ein oder mehrere Wasserstoffatome gegebenenfalls durch Niederalkyl mit 1 bis 3 Kohlenstoffatomen, Niederalkoxy mit 1 bis 3 Kohlenstoffatomen, Halogen oder Trifluormethyl substituiert sind), R-O-CO-, einen Peptidylrest oder für (Peptidylrest)-CO- (wie oben definiertes Niederalkyl) steht;

Q eine Carboxylat-verwandte Gruppe ist;

$R^1$ gegebenenfalls substituiertes Niederalkylen, nämlich eine 2 bis 6 Kohlenstoffatome enthaltende, unverzweigte gesättigte Kohlenwasserstoffkette, worin ein oder mehrere Wasserstoffatome gegebenenfalls durch Niederalkyl mit 1 bis 3 Kohlenstoffatomen, Niederalkoxy mit 1 bis 3 Kohlenstoffatomen, Halogen oder Trifluormethyl substituiert sind, repräsentiert; und

$R^2$, $R^3$ und $R^4$ jeweils unabhängig H oder R (wie oben definiert) bedeuten, mit der Maßgabe, daß wenigstens einer der Reste $R^2$, $R^3$ und $R^4$ nicht Wasserstoff ist,

welches Verfahren umfaßt

(a) die Alkylierung einer Verbindung der Formel:

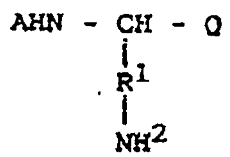

(I)

oder eines funktionellen Derivats davon, in welcher A, Q und $R^1$ wie Vorstehend definiert sind, mit einer Formamidinsulfonsäure der Formel:

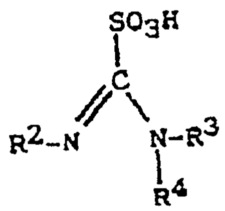

(II)

in welcher

$R^2$, $R^3$ und $R^4$ wie vorstehend definiert sind, und gegebenenfalls

(b) die Umwandlung eines Säureadditionssalzes der Verbindung der Formel (III) mit Base in eine Verbindung der Formel (III) oder ein Salz derselben mit der Base; oder

(c) die Umwandlung eines Salzes der Verbindung der Formel (III) (abgeleitet von einer Base) mit Säure in die freie Verbindung der Formel (III) oder ein Säureadditionssalz davon; oder

(d) die Umwandlung eines Salzen der Verbindung der Formel (III), vorzugsweise eines löslichen Salzes, in ein anderes Salz der Verbindung der Formel (III), vorzugsweise ein weniger lösliches Salz als das lösliche Salz.

2. Verfahren nach Anspruch 1, worin A für H oder R-O-CO- (worin R wie in Anspruch 1 definiert ist) steht.

3. Verfahren nach Anspruch 2, worin A für H steht.

4. Verfahren nach Anspruch 2, worin A für t-Butoxycarbonyl steht.

5. Verfahren nach irgendeinem der Ansprüche 1 bis 4, worin Q für -COOH oder -COOR (worin R wie in Anspruch 1 definiert ist) steht.

6. Verfahren nach irgendeinem der vorangehenden Ansprüche, worin $R^1$ für $-(CH_2)_n-$ steht und n eine ganze Zahl von 3 bis 5 ist.

7. Verfahren nach irgendeinem der vorangehenden Ansprüche, worin jeder der Reste $R^2$ und $R^3$ unabhän-

gig gegebenenfalls substituiertes Niederalkyl (wie in Anspruch 1 definiert) bedeutet und $R^4$ Wasserstoff ist.

8. Verfahren nach irgendeinem der vorangehenden Ansprüche, worin jeder der Reste $R^2$ und $R^3$ gegebenenfalls substituiertes Ethyl bedeutet, worin ein oder mehrere Wasserstoffatome gegebenenfalls durch Niederalkyl mit 1 bis 3 Kohlenstoffatomen, Niederalkoxy mit 1 bis 3 Kohlenstoffatomen, Halogen oder Trifluormethyl substituiert sind.

9. Verfahren nach irgendeinem der Ansprüche 1 bis 6, worin $R^2$ gegebenenfalls substituiertes Phenyl ist, worin ein oder mehrere Wasserstoffatome gegebenenfalls durch Niederalkyl mit 1 bis 3 Kohlenstoffatomen, Niederalkoxy mit 1 bis 3 Kohlenstoffatomen, Halogen oder Trifluormethyl substituiert sind, und $R^3$ und $R^4$ jeweils Wasserstoff bedeuten.

10. Verfahren nach Anspruch 9, worin $R^2$ Phenyl ist.

11. Verfahren nach Anspruch 1, worin die Verbindung der Formel I Lysin ist.

12. Verfahren nach Anspruch 11, worin die Formamidinsulfonsäure der Formel II N, N'-Diethylformamidinsulfonsäure ist.

13. Verfahren nach Anspruch 1, worin A für (Peptidylrest)-CO-(Niederalkyl)- steht, Q -COOH oder -COOR bedeutet und $R^1$ $-(CH_2)_n-$ ist, wobei n eine ganze Zahl von 3 bis 5 bedeutet.

14. Verfahren nach Anspruch 13, worin jeder der Reste $R^2$ und $R^3$ unabhängig für gegebenenfalls substituiertes Niederalkyl steht und $R^4$ Wasserstoff ist.

15. Verfahren nach Anspruch 14, worin jeder der Reste $R^2$ und $R^3$ Ethyl ist.

16. Verfahren nach irgendeinem der Ansprüche 13 bis 15, worin die Verbindung der Formel I (S)-2-[(S)-N-[-(S)-1-Carboxy-5-aminopentyl]-alanyl]-1,2,3,4-tetrahydroisochinolin-3-carbonsäure oder ein Salz derselben oder ein Mono- oder Diester davon ist.

17. Verfahren nach Anspruch 16, wenn abhängig von Anspruch 15, worin (S)-2-[(S)-N-[(S)-1-Carboxy-5-N',N"-diethylguanidinopentyl]alanyl]-1,2,3,4-tetrahydroisochinolin-3-carbonsäure oder ein pharmazeutisch annehmbares, nichttoxisches Salz davon hergestellt wird.

18. Verfahren nach irgendeinem der vorangehenden Ansprüche, welches umfaßt die Herstellung eines $N^G$, $N^{G'}$-Dialkylguanidinopeptids aus einer Verbindung der Formel III.

19. Verfahren nach irgendeinem der vorangehenden Ansprüche, welches umfaßt die Herstellung von ACE-Inhibitoren.

20. Verfahren nach irgendeinem der vorangehenden Ansprüche für die Herstellung von Inhibitoren für das Angiotensin umwandelnde Enzym mit der Formel A

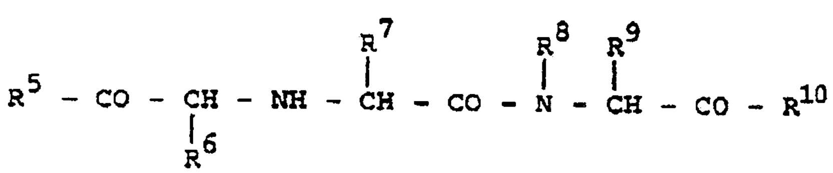

(A)

worin $R^5$ Hydroxy, Niederalkoxy (-OR, worin R Niederalkyl wie unten definiert ist), Benzyloxy oder Amino, gegebenenfalls mit 1 oder 2 Niederalkylgruppen, nämlich 1 bis 6 Kohlenstoffatome enthaltenden verzweigten oder unverzweigten gesättigten Kohlenwasserstoffketten, substituiert, bedeutet;
$R^6$ und $R^7$ repräsentieren
(1) Wasserstoff oder vorzugsweise Niederalkyl (wie oben definiert) (gegebenenfalls omega-substituiert mit Phenyl oder Naphthyl, weniger bevorzugt omega-substituiert mit Di(nieder)alkylguanidino), oder

(2)

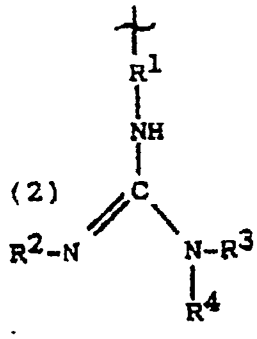

worin $R^1$ gegebenenfalls substituiertes Niederalkylen (wie in Anspruch 1 definiert) ist und $R^2$, $R^3$ und $R^4$ jeweils aus H und R (worin R gegebenenfalls substituiertes Niederalkyl, Phenyl und Aralkyl, immer wie in Anspruch 1 definiert, ist) ausgewählt sind, mit der Maßgabe, daß wenigstens eine der Gruppen $R^2$, $R^3$ und $R^4$ nicht Wasserstoff ist; n eine ganze Zahl von 3 bis 5, vorzugsweise 4 ist; und einer der Reste $R^6$ und $R^7$ für (1) und der andere für (2) steht; und
die Gruppe

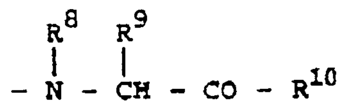

ein Aminosäurerest ist, der ausgewählt ist aus der Gruppe von Iminosäureresten, vorzugsweise cyclischen Iminosäureresten, in welchen die Untergruppe

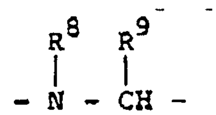

einen heterocyclischen Rest, der ein Stickstoffatom und bis zu 9 Ring-Kohlenstoffatome enthält, bildet, insbesondere wenn dieser cyclische Iminosäurerest für Prolin, Hydroxyprolin oder Prolinanaloga steht oder davon abgeleitet ist;
$R^{10}$ Hydroxy, Niederalkoxy (wie oben definiert), Benzyloxy oder Amino, gegebenenfalls substituiert mit einer oder zwei Niederalkylgruppen (wie oben definiert) darstellt; und der pharmazeutisch annehmbaren, nicht-toxischen Salze davon, welches weiter umfaßt:

(a) die Kondensation eines Aminosäurederivats der Formel

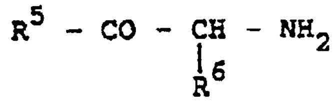

worin $R^6$ die obige Gruppe (2) darstellt,
mit einem $\alpha$-Ketosäurederivat der Formel

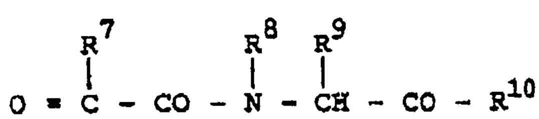

unter reduktiven Bedingungen, worin $R^5$, $R^7$, $R^8$, $R^9$ und $R^{10}$ wie oben definiert sind; oder

(b) die Kondensation eines $\alpha$-Ketosäurederivats der Formel

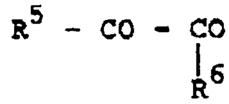

mit einem Peptid der Formel

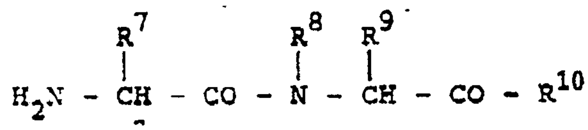

worin $R^7$ für die obige Gruppe (2) steht,
unter reduktiven Bedingungen, worin $R^5$, $R^6$, $R^8$, $R^9$ und $R^{10}$ wie oben definiert sind; oder
(c) die Kondensation eines Aminosäurederivats der Formel

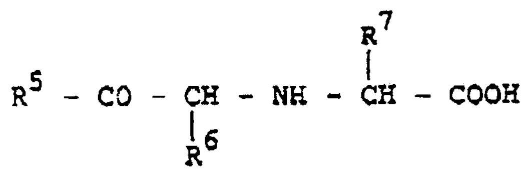

mit Prolin oder einem Prolinanalog-Derivat der Formel

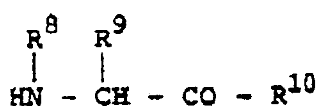

worin $R^5$, $R^6$, $R^7$, $R^8$, $R^9$ und $R^{10}$ wie oben definiert sind; oder
(d) die Alkylierung eines Peptidderivats der Formel

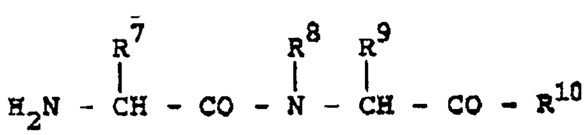

worin $R^7$ für die obige Gruppe (2) steht,
mit einem Alkylierungsmittel der Formel

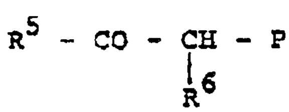

worin P Halogen, ausgewählt aus der Gruppe von Chlor, Brom oder Jod, oder eine Sulfonyloxygruppe ist und $R^5$, $R^6$, $R^8$, $R^9$ und $R^{10}$ wie oben definiert sind; oder
(e) die Alkylierung eines Aminosäurederivats der Formel

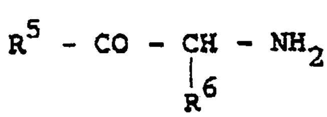

worin $R^6$ für die obige Gruppe (2) steht,
mit einem Alkylierungsmittel der Formel

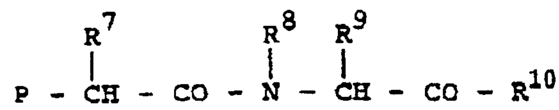

worin P die Bedeutungen von Stufe (e) hat und $R^5$, $R^7$, $R^8$, $R^9$ und $R^{10}$ wie oben definiert sind; oder
(f) die Hydrolyse einer Verbindung der Formel

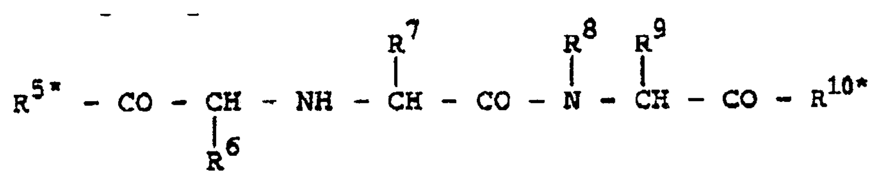

worin $R^{5*}$ und $R^{10*}$ für Hydroxy, Niederalkoxy (wie oben definiert) oder Benzyloxy stehen und wenigstens einer der Reste $R^{5*}$ und $R^{10*}$ Niederalkoxy oder Benzyloxy ist, in eine Verbindung dar Formel A, worin $R^5$ und $R^{10}$ Hydroxy bedeuten; oder
(g) die Hydrogenolyse (reduktive Spaltung) einer Verbindung der Formel

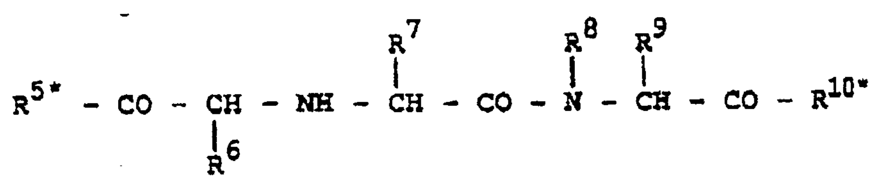

worin $R^{5*}$ und $R^{10*}$ Hydroxy, Niederalkoxy (wie oben definiert) oder Benzyloxy sind und wenigstens einer der Reste $R^{5*}$ und $R^{10*}$ Benzyloxy bedeutet, in eine Verbindung der Formel A, worin wenigstens einer der Reste $R^5$ und $R^{10}$ Hydroxy darstellt; oder
(h) die Umsetzung einer Verbindung der Formel A mit einer Base, um ein Salz der Verbindung der Formel A zu bilden; oder
(i) die Umsetzung einer Verbindung der Formel A mit einer Säure, um ein Säureadditionssalz der Verbindung der Formel A zu bilden; oder
(j) die Veresterung einer Verbindung der Formel

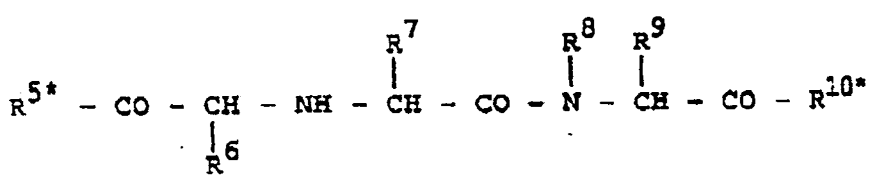

worin wenigstens einer der Reste $R^{5*}$ und $R^{10*}$ Hydroxy darstellt und der andere Rest Hydroxy, Niederalkoxy oder Benzyloxy ist, mit einem Niederalkanol oder Benzylalkohol; oder
(k) die Acylierung von Ammoniak, eines Niederalkylamins oder eine Di(nieder)alkylamins mit einem Dipeptidderivat der Formel

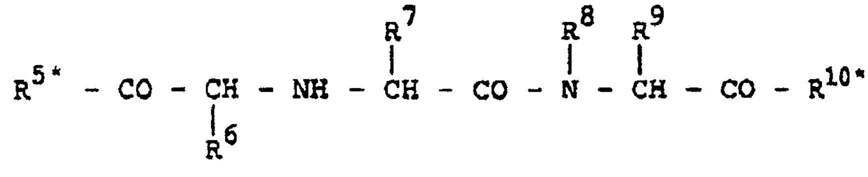

worin $R^{5*}$ und $R^{10*}$ Hydroxy, Chlor, Brom, Niederalkoxy, Benzyloxy, eine Niederalkanoyloxygruppe oder Amino, gegebenenfalls substituiert mit einer oder zwei Niederalkylgruppen, bedeuten und wenigstens einer der Reste $R^{5*}$ und $R^{10*}$ Hydroxy, Chlor, Brom, Niederalkoxy, Benzyloxy oder eine Niederalkanoyloxygruppe ist; und

(l) die Umwandlung eines Säureadditionssalzes des Peptids der Formel A mit Base in ein Peptid der Formel A oder ein Salz davon mit der Base; oder
(m) die Umwandlung eines Salzes eines Peptids der Formel A (abgeleitet von einer Base) mit Säure in das freie Peptid der Formel A oder ein Säureadditionssalz davon; oder
(n) die Umwandlung eines Salzes des Peptids der Formel A, vorzugsweise eines löslichen Salzes, in ein anderes Salz des Peptids der Formel A, vorzugsweise ein weniger lösliches als das lösliche Salz.